# EUROPEAN PATENT APPLICATION

(11) **EP 3 181 132 A1**
(43) Date of publication of application: **21.06.2017**
(21) Application number: 16190916.3
(22) Date of filing: 15.01.2007
(51) Int. Cl.: A61K 31/215, C07C 219/10, A61P 35/00, A61P 17/00

(54) **POSITIVELY CHARGED WATER-SOLUBLE PRODRUGS OF RETINOIDS AND RETINOID-LIKE COMPOUNDS WITH VERY HIGH SKIN PENETRATION RATES**

(62) Divisional of application: 07700593.2
(71) Applicant: Yu, Chongxi, Kensington, MD 20895 (US)
(72) Inventor: Yu, Chongxi, Kensington, MD 20895 (US)
(74) Representative: Finnegan Europe LLP

(57) **Abstract**

The novel positively charged pro-drugs of retinoids and retinoid-like compounds in the general formula (31) 'Structure 31' were designed and synthesized. The compounds of the general formula (31) 'Structure 31' indicated above can be prepared from retinoic acids and related compounds, by reaction with suitable alcohols, thiols, or amines and coupling reagents, such as N, N'-Dicyclohexylcarbodiimide, N,N'-Diisopropylcarbodiimide, O- (Benzotriazol-1-yl)-N,N,N',N'-tetramethyluronium tetrafluoroborate, O- (Benzotriazol-1-yl)-N,N,N',N'-tetramethyluronium hexafluorophosphate, Benzotriazol- 1-yl-oxy-tris(dimethylamino)phosphonium hexafluorophosphate, et al. The positively charged amino groups of these pro-drugs not only largely increases the solubility of the drugs in water, but also bonds to the negative charge on the phosphate head group of membranes. This bonding will disturb the membrane a little bit and may make some room for the lipophilic portion of the prodrug. When the molecules of membrane move, the membrane may 'crack' a little bit due to the bonding of the prodrug. This will let the prodrug insert into the membrane. At pH 7.4, only about 99% of amino group is protonated. When the amino group is not protonated, the bonding between the amino group of the prodrug and the phosphate head group of membrane will disassociate, and the prodrug will enter the membrane completely. When the amino group of the prodrug flips to the other side of the membrane and thus become protonated, then the prodrug is pulled into the cytosol, a semiliquid concentrated aqueous solution or suspension. The results suggest that the pro-drugs diffuse through human skin -350 times faster than do retinoids and retinoid-like compounds. In plasma, more than 90% of these pro-drugs can change back to the parent drugs in a few minutes. The prodrugs can be used medicinally in treating any retinoids and retinoid-like compounds-treatable conditions in humans or animals. The prodrugs can be administered transdermally for any kind of medical treatments and avoid most of the side effects of retinoids and retinoid-like compounds. Controlled transdermal administration systems of the prodrug enables retinoids and retinoid-like compounds to reach constantly optimal therapeutic blood levels to increase effectiveness and reduce the side effects of retinoids and retinoid-like compounds. Another great benefit of transdermal administration of these pro-drugs is that administering medication, especially to children, will be much easier.

## Description

### Technical Field

The present invention relates to the preparations of positively charged and watersoluble pro-drugs of retinoids and retinoid-like compounds and their medicinal use in treating any retinoids and retinoid-like compounds-treatable conditions in humans or animals. More specifically, the present invention is to enable quick skin penetration of retinoids and retinoid-like compounds

### Background Art

Retinoids are a class of compounds consisting of four isoprenoid units joined in a head to tail manner. The retinoids include all-trans-retinoic acid (tretinoin), cis-isomeric retinoic acids, e.g., 13-cis-retinoic acid (isotretinoin) , 9-cis-retinoic acid (alitretinoin), vitamin A (retinol), 4-[1-(5,6,7,8-tetrahydro-3,5,5,8,8-pentamet hyl-2-naphthalenyl) ethenyl]benzoic acid (bexarotene, Targretin ®), retinal, retiferol, (E,E,E)-7-(2-n-propoxy-5,5,8,8-tetramethyl-5,6,7,8-tetrahydro-naphthalen-3-yl)-6-fluo ro-3-methylocta-2,4,6-trienoic acid, adapalene (6-[3-(1-adamantyl)-4-methoxyphenyl]-2-naphthoic acid), acyclic retiniod [(2E, 4E, 6E, 10E)-3, 7, 11, 15-tetramethy-2,4,6,10,14-hexadecapentaenoic acid], ethyl(E,E,E)-7-(2-n-propoxy-5,5,8,8-tetramethyl-5,6,7,8-tetrahydro-naphthalen-3-yl)-6-fluoro-3-methylocta-2,4,6-trienoate, and their derivatives, both natural and synthetic. Various synthetic retinoids and retinoid-like compounds having retinoid activity are described in various patents (U.S. Pat. Nos. 5,648,563; 5,648,385; 5,618,839; 5,559,248; 5,616,712; 5,616,597; 5,602,135; 5,599,819; 5,556,996; 5,534,516; 5,516,904; 5,498,755; 5,470,999; 5,468,879; 5,455,265; 5,451,605; 5,426,118; 5,407,937; 5,399,586; 5,399,561; 5,391,753 ). The retinoids are essential for many of life's processes including vision, reproduction, metabolism, differentiation, bone development, and pattern formation during embryogenesis. Vitamin A (retinol) and retinal are in chemical equilibrium in the body and have equivalent antixe-rophthalmic acitivity. Retinol combines with opsin, the rod pigment in the retina, to form rhodopsin, which is necessary for visual adaptation to darkness. Vitamin A deficiency is characterized by nyctalopia, keratomalacia, keratinization and drying of skin, lowered resistance to infection, retardation of growth, thickening of bone, d iminished production of cortical steroids, and fetal malformations (PDR Generics, 1996, second edition, Medical Economics, Montvale, New Jersey, pg 3094). Topical tretinoin (all-trans-retinoic acid) decreases cohesiveness of follicular epithelial cells with decreased microcomedo formation and stimulates mitotic activity increased turnover of follicular epithelial cells causing extrusion of the comedones. Tretinoin is indicated for topical application in the treatment of acne vulgaris, photoaging, hyper-pigmented macules (liver spot) and premature wrinkles, drug-induced photosensitivity, psoriasis, epidermal wound healing, xerophthalmia, keloids, hyperkeratotic skin disease (PDR Generics, 1996, second edition, Medical Economics, Montvale, New Jersey, pg 2981). Isotretinoin inhibits sebaceous gland function and keratinization. Isotretinoin is indicated for the treatment of severe recalcitrant cystic acne, basal cell carcinoma, cervical cancer, mycosis fungoides (cutaneous T-cell lymphoma), Darier's disease, lamellar ichthyosis, pityriasis rubra pilaris, herpes simplex infections, Grover's disease, lichen planus, refractory rosacea, keratosis palmaris et plantaris, leukoplakia, squamous cell skin cancer, and xeroderma pigmentosum. Alitretinoin (9-*cis*-retinoic acid) is a naturally-occurring endogenous retinoid that binds to and activates all known intracellular retinoid receptor subtypes (RAR, RAR, RAR, RXR, RXR, and RXR). Once activated these receptors function as transcription factors that regulate the expression of genes that control the process of cellular differentiation and proliferation in both normal and neoplastic cells. Alitretinoin inhibits the growth of Kaposi's sarcoma (KS) cells in vitro. Alitretinoin is used for the treatment of Kaposi's sarcoma and myelodysplastic syndromes. Retiferol derivatives are used for the treatment of hyperproliferative skin diseases such as psoriasis, basal cell carcinomas, disorders of keratinization and keratosis, neoplastic diseases, disorders of the sebaceous glands such as acne and seborrhoic dermatitis, the conditions associated with photodamage, the skin damaged through sun exposure, the effects of wrinkling, elastosis and premature ageing (Hilpert, et al., U.S. Pat. No. 6437142). Adapalene is used for the topical treatment of acne vulgaris. Acyclic retiniods are used for prevention of second primary tumors (Yasutoshi Muto, et al., the New England Journal of Medicine, 340, 1046 (1999)).(E,E,E)-7-(2-n-propoxy-5,5,8,8-tetramethyl-5,6,7,8-tetrahydro-naphthalen-3-yl )-6-fluoro-3-methylocta-2,4,6-trienoic acid may be used for the treatment of type 2 diabetes and other metabolic disorders (Deng T, et al., Biol. Pharm. Bull. 28(7), 1192, 2005). Targretin® oral formulation is used for the treatment of cutaneous T-cell lymphoma (CTCL), head and neck carcinoma, systemic Kaposi's sarcoma, lung cancer, ovarian cancer, prostate cancer, and renal cell cancer. Topical Targretin is used for the treatment of cutaneous T-cell lymphoma (CTCL).

One alternative method of administering drugs is topical delivery. Topical drug delivery has several advantages. This method helps to avoid inactivation of a drug caused by first pass metabolism in the liver and gastro-intestinal tract. It can provide local delivery of appropriate concentrations of a drug to the intended site of action without systemic exposure. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Yeager tried to use penetration enhancer to deliver PGE for the treatment of male erectile dysfunction (Yeager, James L. U.S. Pat. No. 6,693,135). Susan Milosovich, et al designed and prepared testosteronyl-4-dimethylaminobutyrate.HCl (TSBH), which has a lipophilic portion and a tertiary amine group that exists in the protonated form at physiological pH. They found that the prodrug (TSBH) diffuses through human skin ∼60 times faster than does the drug (TS) itself [Susan Milosovich, et al., J. Pharm. Sci., 82, 227(1993)1.

### Disclosure of Invention

### Technical Problem

Retinoids and retinoid-like compounds are used to treat a variety of health conditions including acne, photoaging, psoriasis, ichthyosis, hair loss, and various cancers.

Unfortunately, retinoids and retinoid-like compounds are too lipophilic and practically insoluble in water. The membranes are bilayers, with the hydrophilic head groups facing outward into the aqueous regions on either side. Retinoids can enter the lipophilic membrane, but they will stay in the lipophilic layer as part of the membrane due to their similarities and cannot efficiently enter the cytosol on the inside of cell. Owing to their high degree of unsaturation, retinoids are extremely sensitive to UV light, air, and oxidizing agents. After topical application of retinoids, they enter the membrane, but do not go into the inside of a cell. The sunlight, air, or oxidizing agent will induce retinoids' chemical reactions and cause skin redness, burning sensation, peeling, cracking, blistering, or itching. When they are taken orally, the first pass metabolism, which refers to the chemical breakdown of compounds in the liver and gastro-intestinal tract, can destroy and inactivate them in a few minutes. Oral administration of retinoids creates unnecessary systemic exposure and causes many side effects.

### Technical Solution

This invention relates to the design and preparation of novel positively charged pro-drugs of retinoids and retinoid-like compounds and their medicinal use. The prodrugs of 9-cis-retinoic acid (alitretinoin), 13-cis-retinoic acid (isotretinoin), all-trans-retinoic acid (tretinoin), vitamin A (retinol), retiferol, adapalene, acyclic retiniod, and retinoid-like compounds have the general formula (1 to 27) 'Structure 1 to 27':

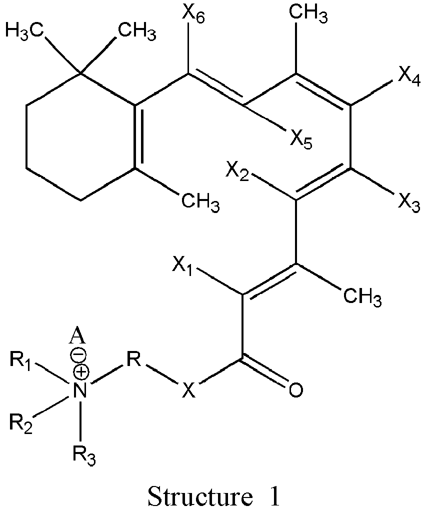

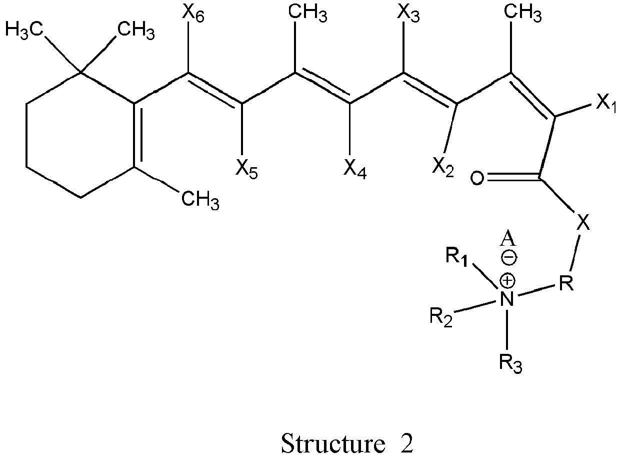

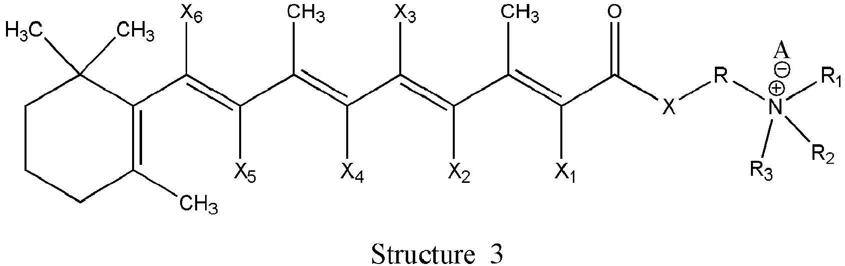

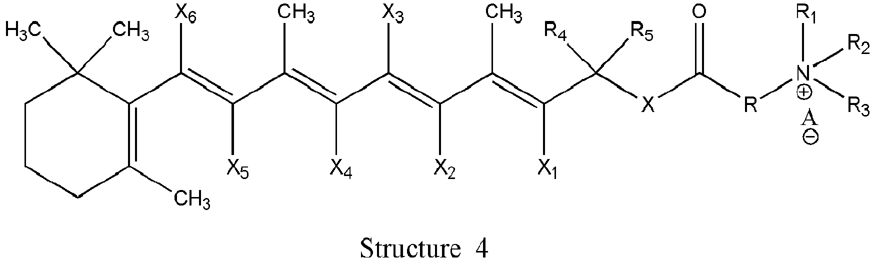

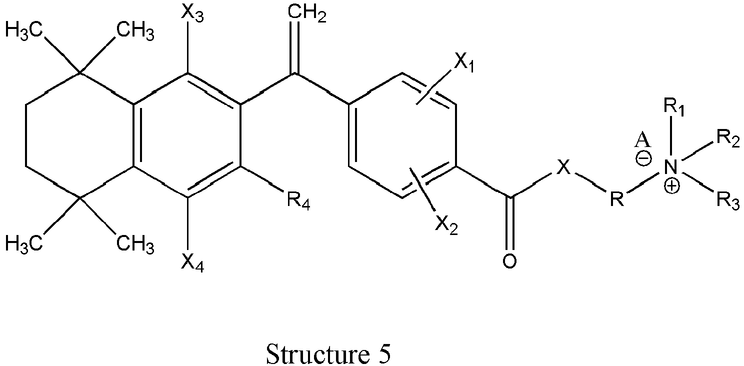

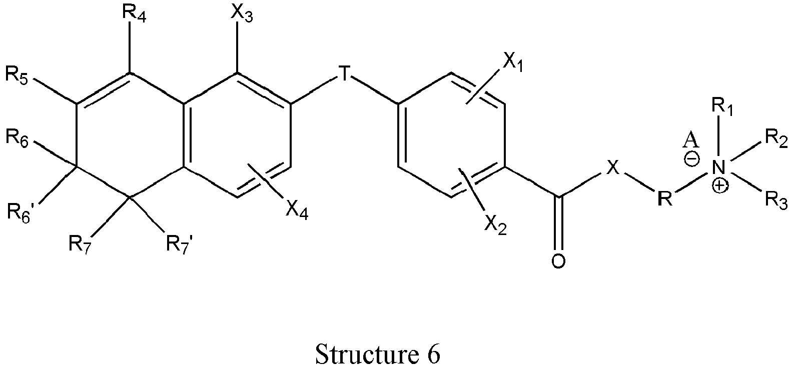

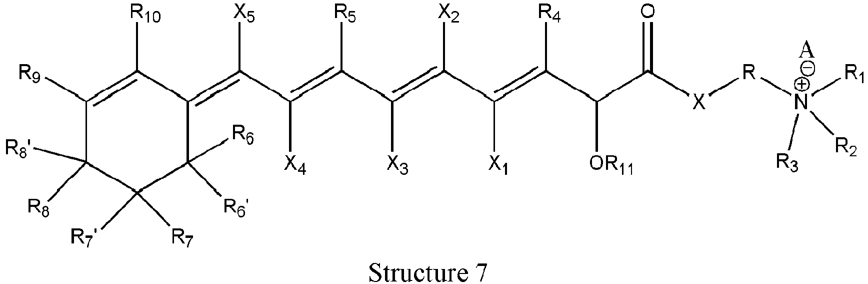

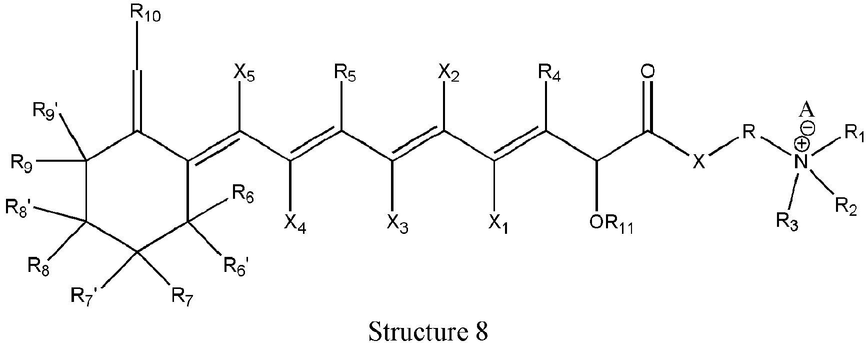

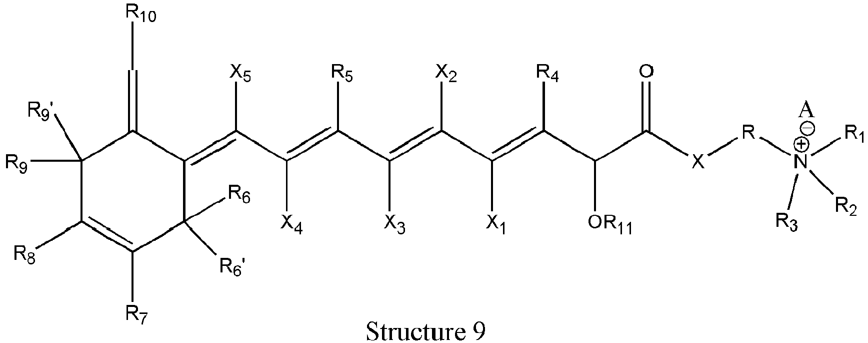

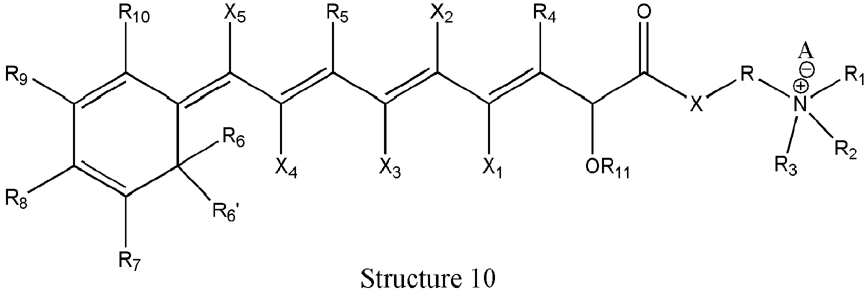

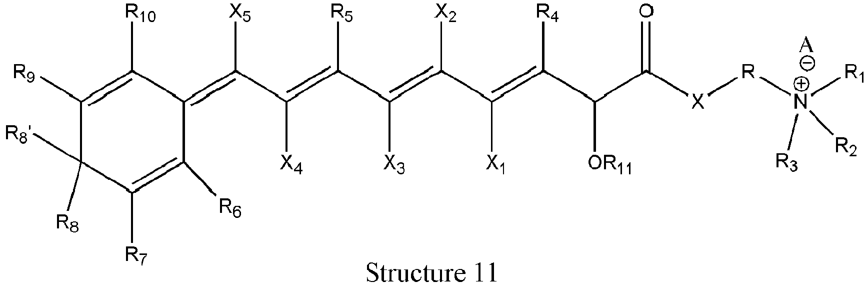

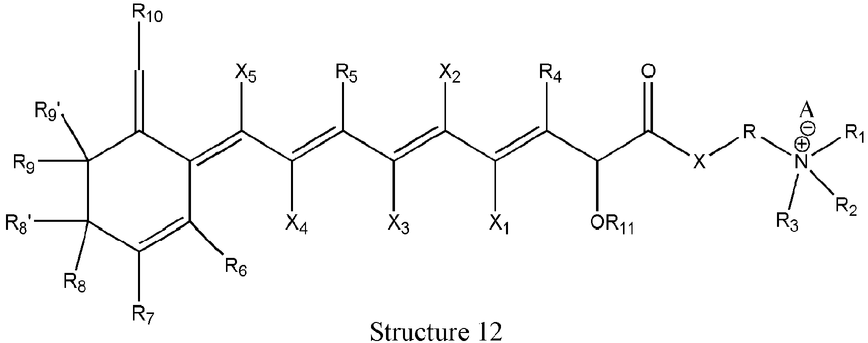

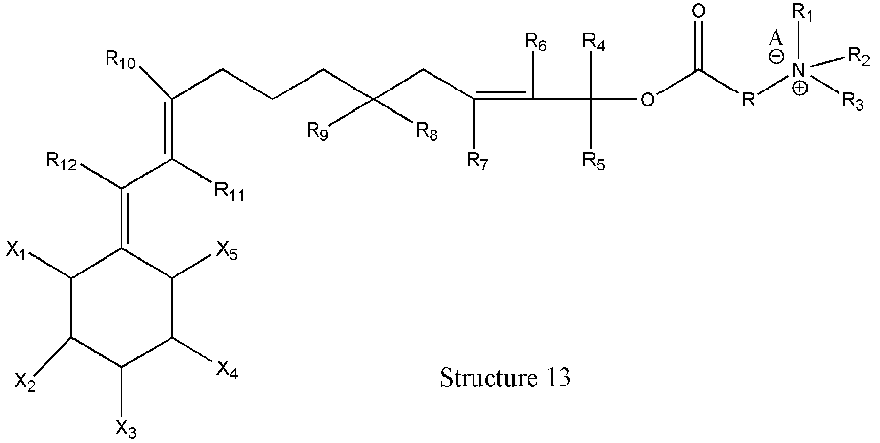

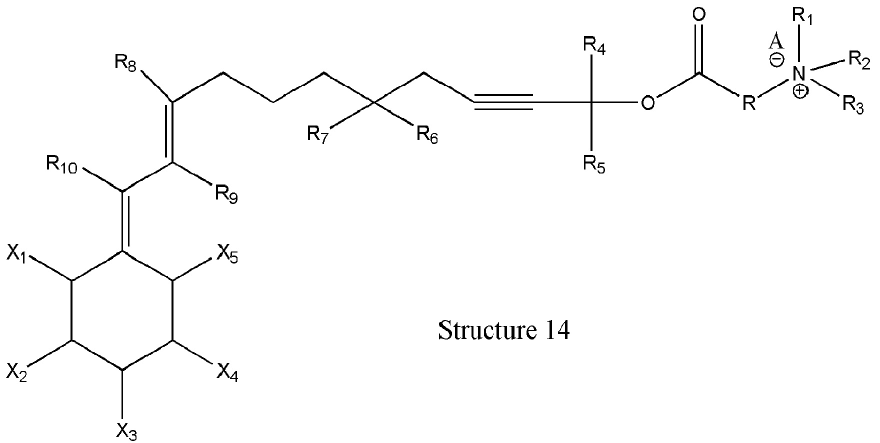

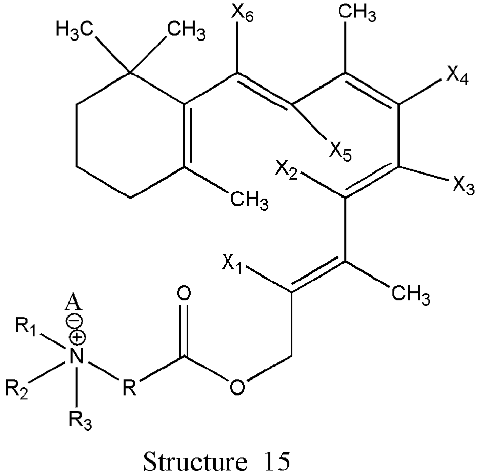

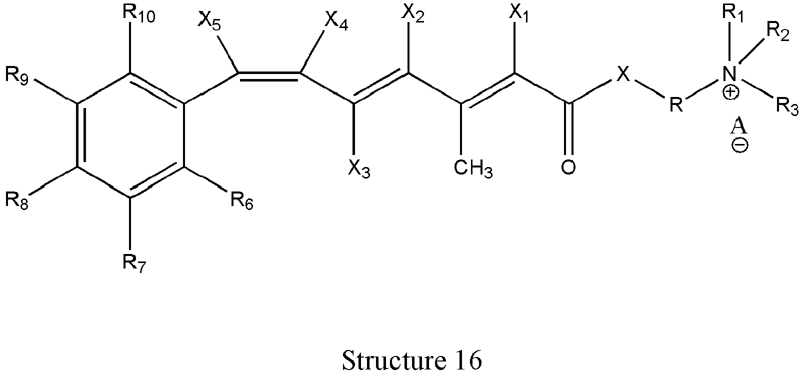

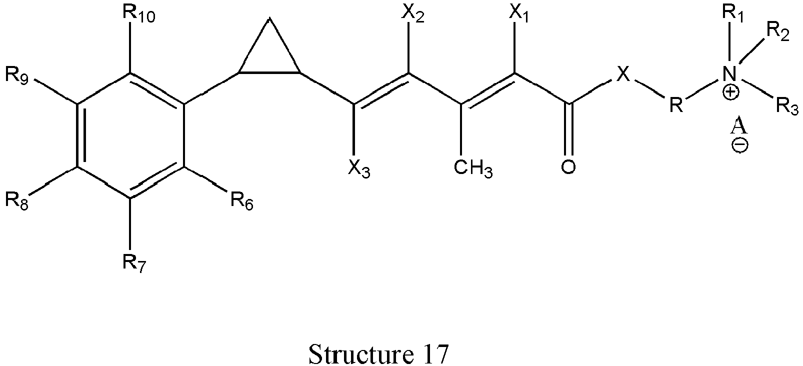

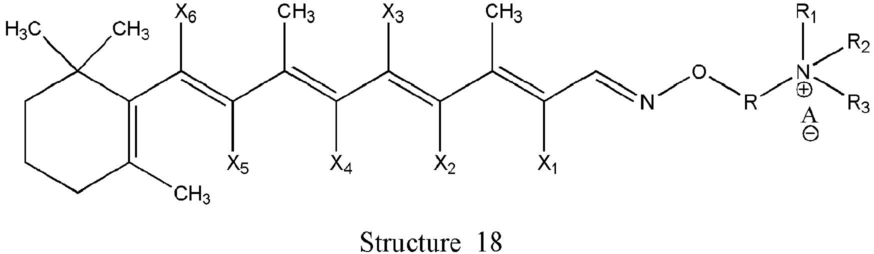

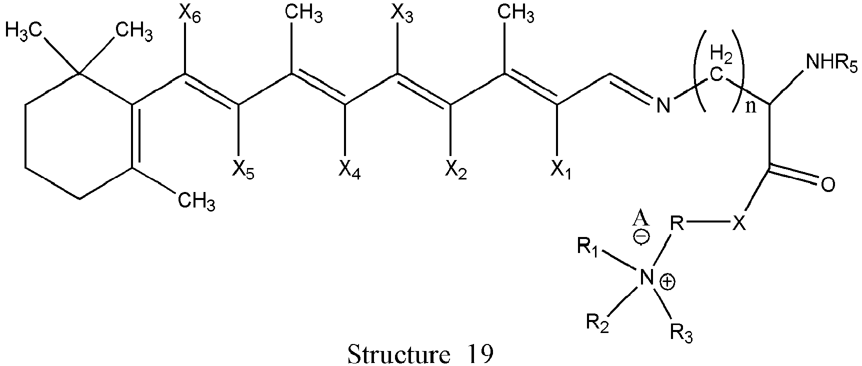

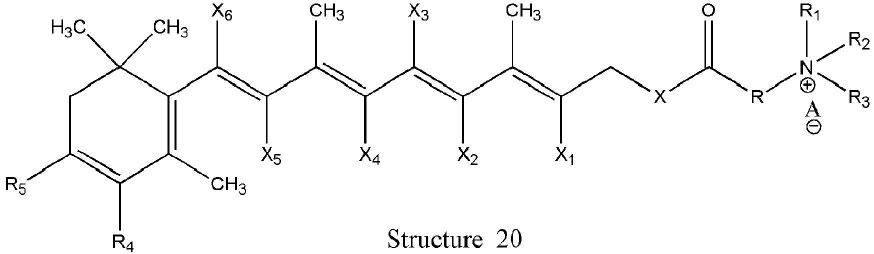

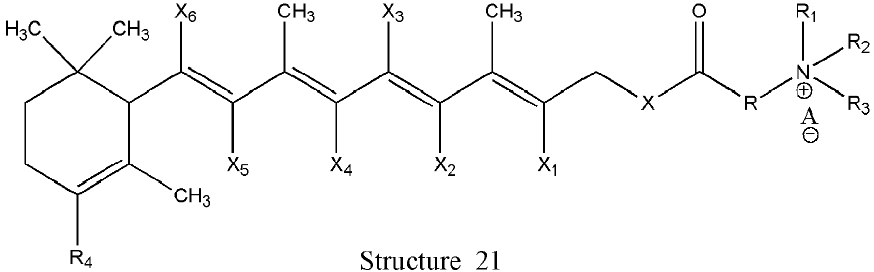

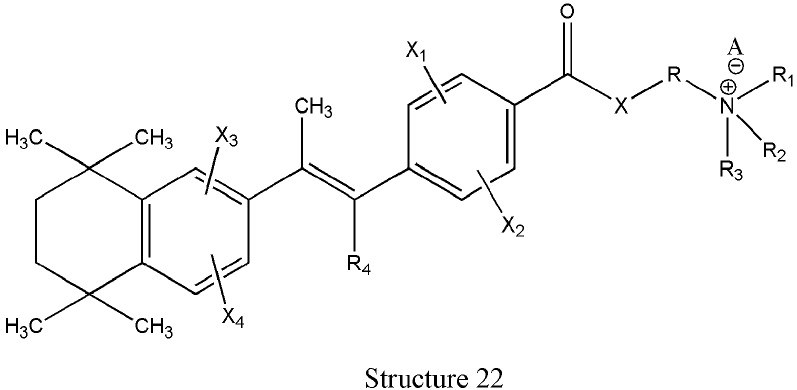

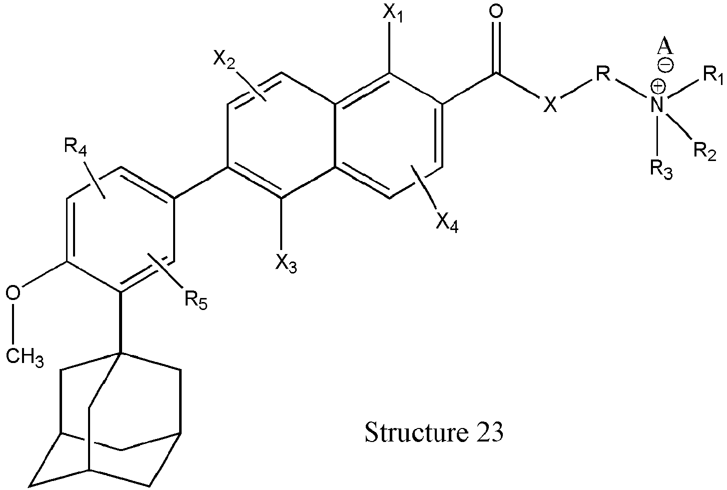

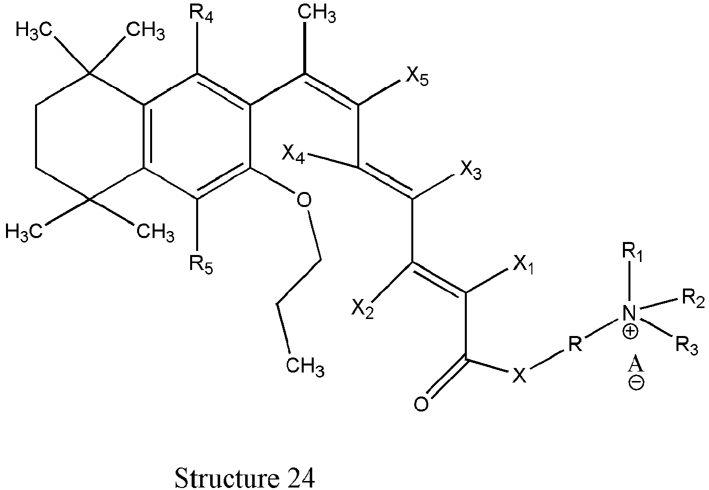

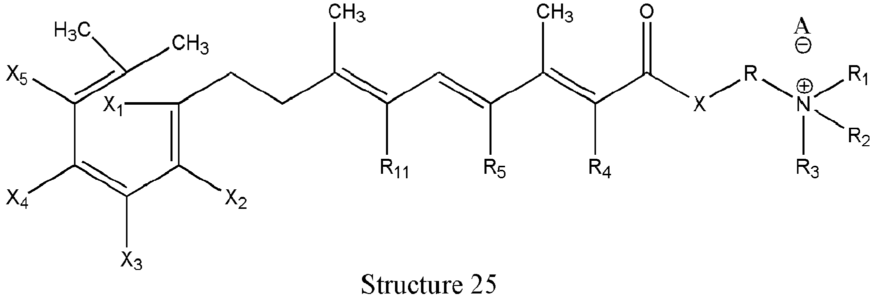

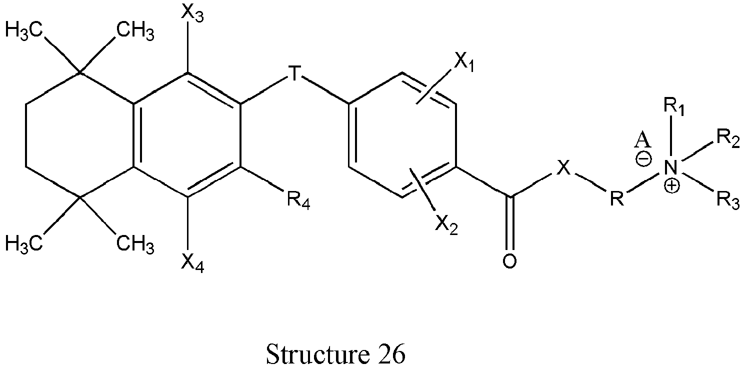

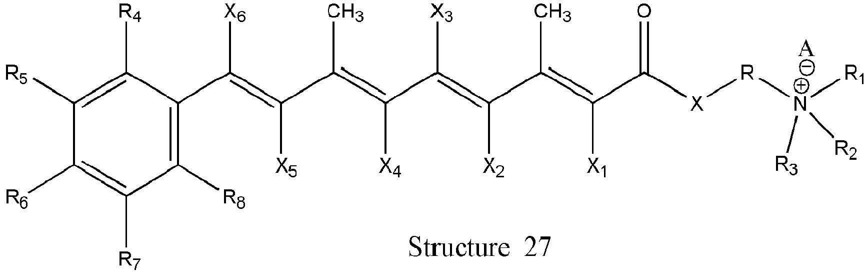

In structure 1-27, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆ R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆ R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO , NO, CN, SO₂R₅, COR₅, COOR₅, NR₄COR ₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₄COR ₅, SOR₅, SR₅, PO₃ R₆ R₆', SOR₅, SR₅ , C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₄COR₅, SOR₅, SR₅, PO₃R₆R₆', SOR₅, SR₅, C₁₋₆ alkyl, C₁₋₆ perfluoroalkyl, C ₁₋₆ alkeny, C₁₋₆ alkyny, or C₁₋₆ alkyloxy; X₄ represents H, OH, Cl, Br, F, I, NO₂, CN, SO ₂R₅, COR₅, COOR₅, NR₄COR₅, SOR₅, SR₅, PO₃ R₆ R₆', SOR₅, SR₅, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₄COR₅, SOR₅, SR₅, PO₂R₆R₆', SOR₅, SR₅, C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂R₅, COR₅, COOR₅, NR₄COR₅, SOR₅, SR₅, PO₃R₆R₆', SOR₅, SR₅, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R'₆, SOR₅, SR₅, C₁₋₆alkyl, C₁₋₆ alkyloxy, C₁₋₆ perfluoroalkyl, C₁₋₆alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₄, SOR₆, SR₆, PO₃R₆R₆',SOR₆, SR₆, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₅, COR₅, COOR₅, NR₆ COR₅, SOR₅, SR₅, PO₃R₆R₆', SOR₅, SR₅, C ₁₋₆ alkyl, C₁₋₆alkyloxy, C₁₋₆ perfluoroalkyl, C₁₋₆ alkeny, C₁₋₆ alkyny, or C₁₋₆ alkyl halide; R₁₂ represents H, OH, Cl, Br, F, I, NO , CN, SO₂ R₅, COR₅, COOR₅, NR₆COR₅, SOR₅, SR₅, PO₃ R₆ R₆', SOR₅, SR₅, C₁₋₆ alkyl, C₁₋₆ alkyloxy, C₁₋₆ perfluoroalkyl, C₁₋₆ alkeny, C ₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇ R₇', SOR₅, SR₅, C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇and R_{7'} taken alone are same or different and are H, Cl, Br, F, I, OH, NO₂, CN, SO₂R₅, COR₅, COOR₅, NR₆COR₅, SOR₅, SR₅, PO₃R₆R₆', SOR₅, SR₅, C ₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆ R₆', aryl or heteroaryl residues, or other ring systems; R₈ and R₈' taken alone are same or different and are H, Cl, Br, F, I, OH, NO₂, CN, SO₂R₅, COR₅, COOR₅, NR₄COR₅, SOR₅, SR₅, PO₃R₆R₆', SOR₅, SR₅, C₁₋₆ alkyl, C ₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₉ and R₉' taken alone are same or different and are H, Cl, Br, F, I, OH, NO₂, CN, SO₂ R₅, COR₅, COOR₅, NR₄COR₅, SOR₅, SR₅, PO₃R₆R₆', SOR₅, SR₅, C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆' , aryl or heteroaryl residues, or other ring systems; R₁₀ and R₁₀' taken alone are same or different and are H, Cl, Br, F, I, OH, NO₂, CN, SO₂R₅, COR₅, COOR_{5,} NR₄COR₅, SOR₅, SR₅, PO₃ R₆R₆', SOR₅, SR₅, C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; T represents, CH₂=C, CH=CH, C(CH₃)=CH, C≡C, C=O, C=S, CONH, CSNH, COO, OCO, COS, COCH₂, or CH₂CO; A⁻ represents Cl⁻, Br⁻, F⁻, I⁻, AcO⁻, citrate, or any negative ions; the double bonds on the side chain may have Z or E configuration; All R, R₁, R₂ R₃, R₄, - (CH₂)ₙ- groups are branched or straight chains, and may contain C, H, O, S, N, and other atoms and may contain single, double, triple bonds and ring systems.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. The membranes are bilayers, with the hydrophilic head groups facing outward into the aqueous regions on either side. Very hydrophilic drugs cannot pass the hydrophobic layer of a membrane and very hydrophobic drugs will stay in the hydrophobic layer as part of the membrane due to their similarities and cannot efficiently enter the cytosol on the inside.

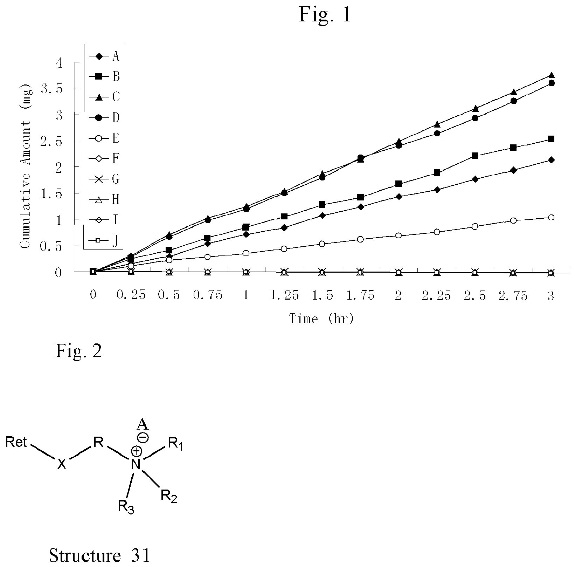

The goal of this invention is to make retinoinds and related compounds administrable transdermally (topical application) by increasing their solubility in the moisture available on the skin surface and their penetration rate through the membrane and skin barrier. These novel pro-drugs of retinoids and related compounds have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs in water. In many instances, the lowest or rate-limiting step in the sequence is the dissolution of the drug. Retinoids and related compounds have a very low solubility in the moisture available on the skin surface, and they will not pass across the barrier of skin in a molecular form efficiently. When they enter the membranes of the skin, they will stay there as part of the membrane due to their similarities and cannot efficiently enter the cytosol, a semi-liquid concentrated aqueous solution or suspension on the inside of the cell. When these new pro-drugs are administered transdermally in a dosage form such as a solution, spray, lotion, ointment, emulsion or gel, they will dissolve in the moisture available on the skin surface immediately. The positive charge on the amino groups of these pro-drugs will bond to the negative charge on the phosphate head group of a membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol. Due to the short stay outside the membranes of the skin, the pro-drugs will not cause burning, pain, itching, or swelling of the skin and the skin will be not sensitive to sun light. The penetration rates of these prodrugs through human skin were measured in vitro by using modified Franz cells, which were isolated from human skin tissue (360-400 µm thick) of the anterior and posterior thigh areas. The receiving fluid consisted of 2 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 5% solution of some of the prodrugs of retinoids or a 5% suspension of retinoids in 0.2mL of the mixture of ethanol and pH 7.4-phosphate buffer (0.2M) (v/v, 70/30) are shown in Figure 1. Apparent flux values of 0.72 mg, 0.85 mg, 1.25 mg, 1.21 mg, 0.35 mg, 0.005 mg, 0.005 mg, 0.005 mg, 0.005 mg, and 0.001 mg/cm²/h were calculated for N,N-diethylaminoethyl 9-cis-retinoate.HBr, N,N-diethylaminoethyl 13-cis-retinoate.HBr, N,N-diethylaminoethyl all-trans-retinoate.HBr, retinyl N,N-dimethyl-2-aminoacetate.HCl, N,N-diethylaminoethyl 4-[1-(5,6,7,8-tetrahydro-3,5,5,8,8-pentamethyl-2-naphthalenyl)ethenyl]benzoate.HCl, 9-cis-retinoic acid (alitretinoin), 13-cis-retinoic acid (isotretinoin), all-trans-retinoic acid (tretinoin), vitamin A (retinol), and bexaroten (Targretin^{®}) respectively diffusing through human skin. The pro-drugs diffuse through human skin more than 350 times faster than do retinoids. The results suggest that the positive charge on the dialkyaminoethyl group has a very important role in the passage of the drug across the membrane and skin barrier.

Irritative effect or discomfort in the skin of mice of the novel prodrugs was evaluated during a period of 1 week after the topical application of 0.1 ml of 1 % of the respective test drug in ethanol to the back of nude mice twice perday. None of any signs of irritative effect or discomfort was observed for N,N-diethylaminoethyl 9-cis-retinoate.HBr, N,N-diethylaminoethyl 13-cis-retinoate.HBr, N,N-diethylaminoethyl all-trans-retinoate.HBr, retinyl N,N-dimethyl-2-aminoacetate.HCl, N,N-diethylaminoethyl 4-[1-(5,6,7,8-tetrahydro-3,5,5,8,8-pentamethyl-2-naphthalenyl)ethenyl]benzoate.HCl.

A good prodrug should change back to the parent drug easily. In vitro plasma hydrolysis studies were carried out as the following. 10 mg of the prodrug was dissolved in 0.1 ml of 0.2M pH 7.4 phosphate buffer. 1 ml of human plasma, preheated to 37°C, was added into the mixture. The mixture was kept in a water bath at 37°C. At every 2 min intervals, 0.2 ml of samples were withdrawn and added to 0.4 ml of methanol to precipitate the plasma protein. The samples were centrifuged for 5 min and analyzed by HPLC. The half lives of hydrolysis are 10 min+/-1 min. for N,N-diethylaminoethyl 9-cis-retinoate.HBr, 8 min+/-2 min. for N,N-diethylaminoethyl 13-cis-retinoate.HBr, 9 min+/-1 min. for N,N-diethylaminoethyl all-trans-retinoate.HBr, 13 min+/-2 min. for retinyl N,N-dimethyl-2-aminoacetate.HCl, and 11 min.+/-2 min. for N,N-diethylaminoethyl 4-[1-(5,6,7,8-tetrahydro-3,5,5,8,8-pentamethyl-2-naphthalenyl)ethenyl]benzoate.HCl.

Targretin® (bexarotene) selectively activates a subclass of retinoid receptors called RXRs, which play an important role in several cellular activities. One of the most important of these activities is called programmed cell death, or 'apoptosis', a natural process by which the body rids itself of unwanted cells. Targretin® is being developed by Ligand in both topical and oral formulations. Topical Targretin is used for the treatment of cutaneous T-cell lymphoma (CTCL). In addition, Targretin oral formulation is used for the treatment of CTCL , head and neck carcinoma, systemic Kaposi's sarcoma, lung cancer, ovarian cancer, prostate cancer, and renal cell cancer. Alitretinoin (9-*cis*-retinoic acid) is a naturally-occurring endogenous retinoid that binds to and activates all known intracellular retinoid receptor subtypes (RARa , RARb , RARg , RXRa, RXRb and RXRg). Once activated these receptors function as transcription factors that regulate the expression of genes that control the process of cellular differentiation and proliferation in both normal and neoplastic cells. Al-itretinoid is used for treatment of Kaposi's Sarcoma, AI DS-Related Kaposi's Sarcoma, other skin cancer, breast cancer, and other cancers.

For evaluation of anti-tumor activity of these prodrugs, human breast cancer cells (BCAP-37, 3-4 mm³ of tumor tissue was used in each mouse) were subcutaneous xenografted into nude mice (BALB). After 1 days, 50 µl of 1 % N,N-diethylaminoethyl 9-cis-retinoate .HBr and N,N-diethylaminoethyl 4-[1-(5,6,7,8-tetrahydro-3,5,5,8,8-pentamethyl-2-naphthalenyl)ethenyl]benzoate.HCl in ethanol/0.2M pH 7.4 phosphate buffer (v/v, 70/30) was topically applied to the human breast cancer cells-implanted area (near the front leg) twice perday. After 28 days, the control group (n=7) demonstrated 100% incidence (the average tumor size was 13±2 mm x 12±2 cm), but none of tumor was seen in the test groups (n=7) that treated with N,N-diethylaminoethyl 9-cis-retinoate .HBr or N,N-diethylaminoethyl 4-[1-(5,6,7,8-tetrahydro-3,5,5,8,8-pentamethyl-2-naphthalenyl)ethenyl]benzoate.HCl. The most important thing is that mice that were given the drug did not show any discomfort or irritative effect. The average weight of the treated group is 25±2 grams and that of the control group is 23±3 grams. The results show that these prodrugs have very mild side effects.

In another experiment, human colon cancer cells (LS174J, 3-4 mm³ of tumor tissue was used in each mouse) were subcutaneous xenografted into nude mice (BALB). After 1 days, 50 µl of 1 % N,N-diethylaminoethyl 9-cis-retinoate.HBr and N,N-diethylaminoethyl 4-[1-(5,6,7,8-tetrahydro-3,5,5,8,8-pentamethyl-2-naphthalenyl)ethenyl]benzoate.HCl in ethanol/0.2M pH 7.4 phosphate buffer (v/v, 70/30) was topically applied to the human colon cancer cells-implanted area (near the front leg) twice perday. After 28 days, the control group (n=7) demonstrated 100% incidence (the average tumor size was 22±4 mm x 20±3 mm), but none of tumor was seen in the test groups (n=7) that treated with N,N-diethylaminoethyl 9-cis-retinoate .HBr or N,N-diethylaminoethyl 4-[1-(5,6,7,8-tetrahydro-3,5,5,8,8-pentamethyl-2-naphthalenyl)ethenyl]benzoate.HCl.

The retinoids are all commercially available. The compounds of the general formula (1, 2, 3, 5, 6, 7, 8, 9, 10 , 11, 12, 16, 17, 22, 23, 24, 25, 26, or 27) 'Structure 1, 2, 3, 5, 6, 7, 8, 9, 10 , 11, 12 , 16, 17, 22, 23, 24, 25, 26, or 27' indicated above can be prepared from retinoic acids or related compounds, by reaction with compounds of the general formula (28) 'Structure 28' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris(dimethylamino)phosphonium hexafluorophosphate, et al. 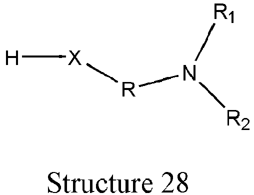 wherein, R represents a branched or straight chain, -(CH₂)ₙ-, wherein n=0, 1, 2, 3, 4, 5, 6, 7, 8, 9, 10 ..., in -(CH₂)ₙ- any CH₂ may be replaced with O, S, CH=CH, C≡C, CR₆R ₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; X represents O, S, or NH.

The compounds of the general formula (4, 13, 14, 15, 20, or 21) 'Structure 4, 13, 14, 15, 20, or 21' indicated above can be prepared from retinol and related compounds, by reaction with compounds of the general formula (29) 'Structure 29'. 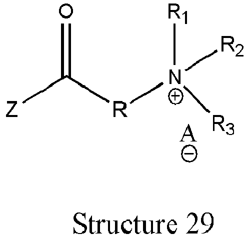 wherein, R represents a branched or straight chain, -(CH₂)ₙ-, wherein n=0, 1, 2, 3, 4, 5, 6, 7, 8, 9, 10 ..., wherein -(CH₂)ₙ-, any CH₂ can be replaced with O, S, CH=CH, C≡C, CR₆ R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C=C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; Z represents F, Cl, Br, or I; A⁻ represents Cl⁻, Br⁻, F⁻, I⁻, AcO⁻, citrate, or any negative ions.

When X represents O, the compounds of the general formula (1, 2, 3, 5, 6, 7, 8, 9, 10, 11, 12, 16, 17, 22, 23, 24, 25, 26, or 27) 'Structure 1, 2, 3, 5, 6, 7, 8, 9, 10, 11, 12, 16, 17, 22, 23, 24, 25, 26, or 27' indicated above can be prepared from metal salts, organic base salts , or immobilized base salts of retinoic acids or related compounds, by reaction with compounds of the general formula (30) 'Structure 30'. 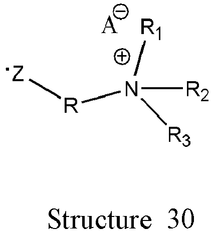 wherein, R represents a branched or straight chain, -(CH₂)ₙ-, wherein n=0, 1, 2, 3, 4, 5, 6, 7, 8, 9, 10, ..., wherein -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; Z represents F, Cl, Br, I, or p-toluenesulphonyl, A represents Cl⁻, Br⁻, F⁻, I⁻, AcO⁻, citrate, or any negative ions.

### Advantageous Effects

These pro-drugs of retinoids and retinoid-like compounds in the present invention have a lipophilic portion and a hydrophilic portion (the amine groups that exist in the protonated form at physiological pH. The positively charged amino groups of these pro-drugs have two major advantages. First, it largely increases the solubility of the drugs in water; when these new pro-drugs are administered transdermally in a dosage form such as a solution, spray, lotion, ointment, emulsion or gel, they will mix with moisture on the skin, eye, genital area, mouth, nose, or other part of the body immediately. Second, the positive charge on the amino group of these pro-drugs will bond to the negative charge on the phosphate head group of the membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay on the skin, eye, genital area, mouth, nose, or other part of the body, the pro-drugs will not cause itching, burning or pain. Experiment results show that more than 90% of the pro-drugs were changed back to the parent drugs in a few minutes. The pro-drugs have a much better absorption rate and as transdermal administration avoids the first pass metabolism, the pro-drugs will be stronger than retinoids and retinoid-like compounds at the same dosage. Another great benefit of transdermal administration of these pro-drugs is that administering medication, especially to children, will be much easier.

### Description of Drawings

Figure 1: Cumulative amounts of N,N-diethylaminoethyl 9-cis-retinoate.HBr (5% solution, A), N,N-diethylaminoethyl 13-cis-retinoate.HBr (5% solution, B), N,N-diethylaminoethyl all-trans-retinoate.HBr (5% solution, C), retinyl N,N-dimethyl-2-aminoacetate.HCl (5% solution, D), N,N-diethylaminoethyl 4-[1-(5,6,7,8-tetrahydro-3,5,5,8,8-pentamethyl-2-naphthalenyl)ethenyl]benzoate.HCl (5% solution, E), 9-cis-retinoic acid (5% suspention, F), 13-cis-retinoic acid (5% suspention, G), all-trans-retinoic acid (5% suspention, H), vitamin A (5% suspention, I), and bexaroten (5% suspention, J), crossing isolated human skin tissue in Franz cells (n=5). In each case, the vehicle was a ethanol/pH 7.4 phosphate buffer (0.2 M) (v/v, 70/30).
Figure 2: Structure 31, wherein, Ret represents retinoids and retinoid-like compounds; R represents a branched or straight chain, -(CH₂)ₙ-, wherein n=0, 1, 2, 3, 4, 5, 6, 7, 8, 9, 10 ..., aryl residues or heteroaryl residues; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ can be replaced with O, S, CH=CH, C≡C, CR₄R₃, aryl or heteroaryl residues, or other ring systems; R₃ represents H, one of any alkyl, alkyloxy, alkenyl or alkynyl residues having 1 to 12 carbon atoms, or aryl and heteroaryl residues; R₄ represents H, one of any alkyl, alkyloxy, alkenyl or alkynyl residues having 1 to 12 carbon atoms, or aryl and heteroaryl residues; X represents O, S, or NH; A⁻ represents Cl⁻, Br⁻, F⁻, I⁻, AcO⁻, citrate, or any negative ions; All R, R₁, R₂, R₃, or -(CH₂)ₙ- groups are branched or straight chains and may include C, H, O, S, or N atoms and may have single, double, and triple bonds.

### Best Mode

### Preparation of N,N-diethylaminoethyl 9-cis-retinoate.HBr.

32.2 g (0.1 mol) of sodium 9-cis-retinoate was dissolved in 100 ml of acetonitrile. 26.1 g (0.1 mol) of 2-Bromo-N,N-diethylethylamine.HBr was added into the reaction mixture. The mixture was stirred for overnight at RT. The solvents were evaporated off. 200 ml of ethanol is added into the residue. The solid is removed by filtration. The solution is evaporated to dryness. 100 ml of ethyl acetate was added into the reaction mixture. Hexane (100 ml) was added. The solid product was collected by filtration. After drying, it yielded 36 g of the desired product (75%). Hygroscopic product. Elementary analysis: C₂₆ H₄₂ BrNO₂; MW: 480.52. Calculated % C: 64.99; H: 8.81; Br: 16.63; N: 2.91; O: 6.66; Found % C: 65.03; H: 8.80; Br: 16.60; N: 2.89; O: 6.68.

### Mode for Invention

### Preparation of N,N-diethylaminoethyl 13-cis-retinoate.HBr.

32.2 g (0.1 mol) of sodium 13-cis-retinoate was dissolved in 100 ml of acetonitrile. 26.1 g (0.1 mol) of 2-Bromo-N,N-diethylethylamine.HBr was added into the reaction mixture. The mixture was stirred for overnight at RT. The solvents were evaporated off. 200 ml of ethanol is added into the residue. The solid is removed by filtration. The solution is evaporated to dryness. 100 ml of ethyl acetate was added into the reaction mixture. Hexane (100 ml) was added. The solid product was collected by filtration. After drying, it yielded 38 g of the desired product (79.1 %). Hygroscopic product. Elementary analysis: C₂₆H₄₂ BrNO₂; MW: 480.52. Calculated % C: 64.99; H: 8.81; Br: 16.63; N: 2.91; O: 6.66; Found % C: 65.03; H: 8.80; Br: 16.60; N: 2.89; O: 6.68.

### Preparation of N,N-diethylaminoethyl all-trans-retinoate.HBr.

32.2 g (0.1 mol) of sodium all-trans-retinoate was dissolved in 100 ml of acetonitrile. 26.1 g (0.1 mol) of 2-Bromo-N,N-diethylethylamine.HBr was added into the reaction mixture. The mixture was stirred for overnight at RT. The solvents were evaporated off. 200 ml of ethanol is added into the residue. The solid is removed by filtration. The solution is evaporated to dryness. 100 ml of ethyl acetate was added into the reaction mixture. Hexane (100 ml) was added. The solid product was collected by filtration. After drying, it yielded 35 g of the desired product (72.9%). Hygroscopic product. Elementary analysis: C₂₆H₄₂BrNO₂; MW: 480.52. Calculated % C: 64.99; H: 8.81; Br: 16.63; N: 2.91; O: 6.66; Found % C: 65.03; H: 8.80; Br: 16.60; N: 2.89; O: 6.68.

### Preparation of retinyl N,N-dimethyl-2-aminoacetate.HCl.

28.6 g (0.1 mol) of retinol was dissolved in 300 ml of acetonitril. 25 ml of triethylamine was added into the reaction mixture. 16 g of N,N-dimethylaminoacetyl chloride hydrochloride was added into the reaction mixture. The mixture was stirred for 5 h at RT. The solid was removed by filtration. The solution was evaporated to dryness. 500 ml of ethyl acetate was added into the residue. 200 ml of 5% of sodium c arbonate solution was added into the mixture with stirring. The organic solution is collected and washed with water (After drying, it yielded 31 g of the desired product (75.5%). Hygroscopic product; Elementary analysis: C₂₄H₃₈ClNO₂; MW: 408.02. Calculated % C: 70.65; H: 9.39; Cl: 8.69; N: 3.43; O: 7.84; Found % C:70.60; H: 9.46; Cl: 8.71; N: 3.42; O: 7.81.

### Preparation of N,N-diethylaminoethyl

### 4-[1-(5,6,7,8-tetrahydro-3,5,5,8,8-pentamethyl-2-naphthalenyl)ethenyl]benzoate.HCl

34.9 g (0.1 mol) of 4-[1-(5,6,7,8 -tetrahydro- 3,5,5,8,8-pentamethyl - 2-naphthalenyl) ethenyl] benzoic acid (bexarotene, Targretin ®) was dissolved in 300 ml of chloroform. 20.6 g of N, N'-Dicyclohexylcarbodiimide was added into the reaction mixture. 11.6 g of dimethylaminoethanol was added into the reaction mixture. The mixture was stirred for 3 hours at RT. The solid was removed by filtration. The chloroform solution was washed with 5% NaHCO₃ (2 x 100 ml) and water (3 x 100 ml). The organic solution was dried over anhydrous sodium sulfate. Sodium sulfate was removed by filtration. 3.6 g of HCl gas in ether (100 ml) was added into the reaction mixture with stirring. The solid product was collected by filtration. After drying, it yielded 40 g of the desired product (85.8%). Hygroscopic product; Elementary analysis: C₃₀H₄₂ ClNO₂; MW: 484.11. Calculated % C: 74.43; H: 8.74; Cl: 7.32; N: 2.89; O: 6.61; Found % C: 74.39; H: 8.76; Cl: 7.29; N: 2.91, O: 6.65.

### Industrial Applicability

The pro-drugs of the general formula (1-27) 'Structure 1-27' are superior to retinoids and retinoid-like compounds. They can be used medicinally in treating any retinoids and retinoid-like compounds-treatable conditions in humans or animals. They may be used for the treatment of acne, acne scarring, psoriasis, ichthyosis, eczema, keratinization disorders, precancerous lesions, chemoprophylaxis, warts, sarcoidosis, treating photoaged skin, preventing photoaged skin, treating chronologically aged skin, hair loss, and various cancers.

The present invention provides:
[1] A compound corresponding to the general formula (1) 'Structure 1', 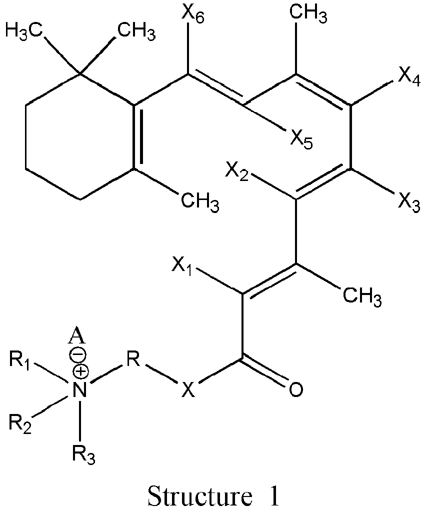 wherein,R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C=C, CR₆ R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ can be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ can be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆ COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR _{5'}SR₅, PO₃ R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR ₆COR₅, SOR₅, SR₅, PO₃ R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅ , COOR₅, NR COR₆, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃ R₆R₆', C₁₋₆ alkyl, C₁₋₆ alkyloxy, C₁₋₆ perfluoroalkyl, C₁₋₆ alkeny, C₁₋₆ alkyny, or C ₁₋₆alkyl halide; R₅ represents H, OH, Cl, Br, F, I, NO , CN, SO₂R₆, COR₆, COOR ₆, NR₆ COR₇, SOR₆, SR₆, PO₃ R₆ R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂ R₅, COR₅, COOR₅, NR₇ COR₅ , SOR₅, SR₅, PO₃R₇R₇', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇' aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅ , SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[2] A compound as in 1, which is N,N-diethylaminoethyl 9-cis-retinoate.HA, wherein, A⁻ represents Cl⁻, Br⁻, F⁻, I⁻, AcO⁻, citrate, or any negative ions.
[3] A compound corresponding to the general formula (2) 'Structure 2', 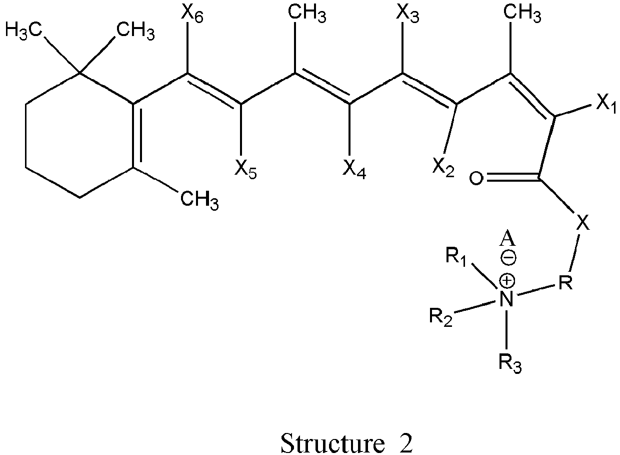 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆ R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆ aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆ R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X represents H, OH, Cl, Br, F, I, NO₂, CN, SO₂R₅, COR₅, COOR₅, NR₆COR₅, SOR ₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR ₆COR₅, PO₃R₆R₆', C₁₋₆alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO R , COR₅, COOR₅, NR ₅COR₅, SOR₅, SR₅, PO₃R₆R₆',C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂ R₅, COR₅, COOR₅, NR₆ COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂ R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₅ represents H, OH, Cl, Br, F, I, NO₂, CN, SO₂R₆, COR₆, COOR₆, NR₆COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇ COR₅, SOR₅, SR₅, PO₃R₇R₇', C₁₋₆ alkyl, C ₁₋₆alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇ and R₇' taken alone are same or different and are H, Cl, Br, F, I, OH, NO₂, CN, SO₂R₅, COR₅, COOR₅, NR₆ COR₅, SOR₅, SR₅, PO₃R₆R₆', C₁₋₆ alkyl, C₁₋₆ alkyloxy, C₁₋₆alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆ R₆', aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R ₁, R₂, R₃, R₄, -(CH₂)ₙ- groups are branched or straight chains, and may contain C, H, O, S, N, and other atoms and may contain single, double, triple bonds and ring systems.
[4] A compound as in 3, which is N,N-diethylaminoethyl 13-cis-retinoate.HA, wherein, A⁻ represents Cl⁻, Br⁻, F⁻, I⁻, AcO⁻, citrate, or any negative ions.
[5] A compound corresponding to the general formula (3) 'Structure 3', 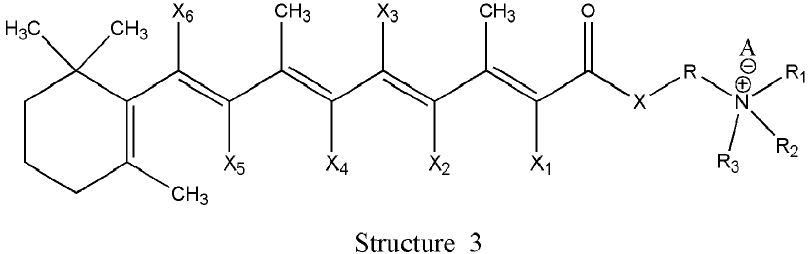 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆',aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR ₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X represents H, OH, Cl, Br, F, I, NO₂, CN, SO₂R₅, COR₅, COOR₅, NR ₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ per-fluoroalkyl, C₁₋₆ alkeny, C₁₋₆ alkyny, or C₁₋₆ alkyloxy; X₆ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₅ represents H, OH, Cl, Br, F, I, NO₂, CN, SO₂R₆, COR₆, COOR₆, NR₆COR₇, SOR₆, SR₆, PO₃ R₆R₆', C₁₋₆ alkyl, C₁₋₆ alkyloxy, C₁₋₆ perfluoroalkyl, C₁₋₆ alkeny, C₁₋₆ alkyny, or C ₁₋₆alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R ₁, R₂, R₃, R₄, -(CH₂)ₙ- groups are branched or straight chains, and may contain C, H, O, S, N, and other atoms and may contain single, double, triple bonds and ring systems.
[6] A compound as in 5, which is N,N-diethylaminoethyl all-trans-retinoate.HA, wherein, A⁻ represents Cl⁻, Br⁻, F⁻, I⁻, AcO⁻, citrate, or any negative ions.
[7] A compound corresponding to general formula (4) 'Structure 4', 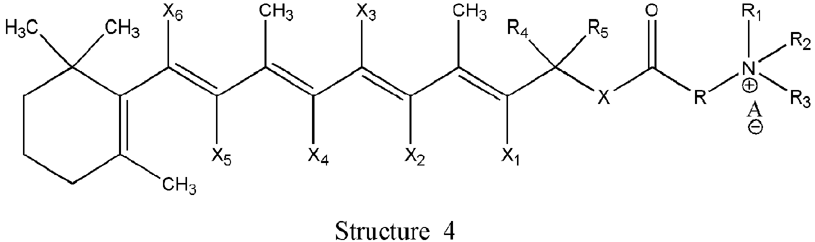 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; R₄ represents H, OH, Cl, Br, F, I, NO₂, CN, SO₂R ₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₇, SOR₆, SR₆, PO₃R₆R₆', C ₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C ₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃ R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR ₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ per-fluoroalkyl, C₁₋₆ alkeny, C₁₋₆ alkyny, or C₁₋₆ alkyloxy; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[8] A compound as in 7, which is retinyl N,N-dimethyl-2-aminoacetate.HA, wherein, A⁻ represents Cl⁻, Br⁻, F⁻, I⁻, AcO⁻, citrate, or any negative ions.
[9] A compound corresponding to the general formula (5) 'Structure 5', 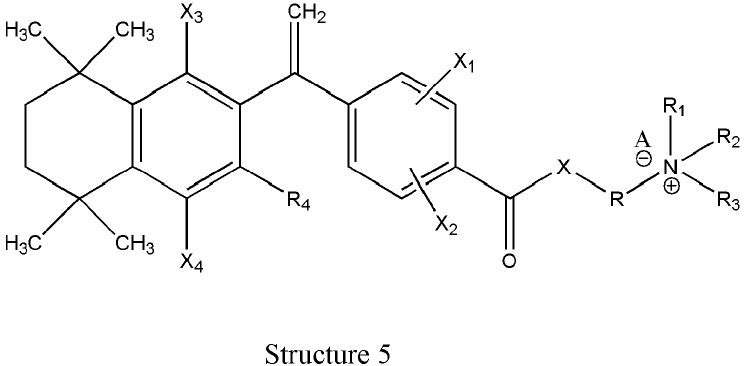 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ can be replaced with O, S, CH=CH, C≡C, CR₆R₆', aryl or heteroaryl residues, or other ring systems; R4 represents H, OH, Cl, Br, F, I, NO₂, CN, SO₂R ₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆, COR₇, SOR₆, SR₆, PO₃R₆R₆', C ₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C ₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃ R₆ R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR ₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; A represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[10] A compound as in 9, which is N,N-diethylaminoethyl 4-[1-(5,6,7,8-tetrahydro-3,5,5,8,8-pentamethyl-2-naphthalenyl)ethenyl]benzoate. H A, wherein, A represents Cl⁻, Br⁻, F⁻, I⁻, AcO⁻, citrate, or any negative ions.
[11] A compound corresponding to general formula (6) 'Structure 6', 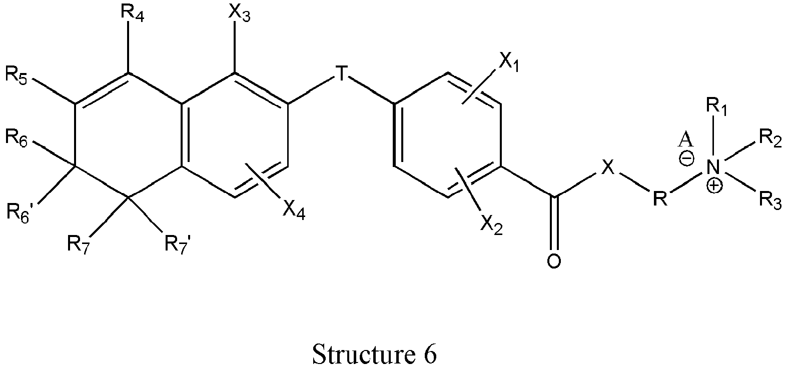 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR ₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR ₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₇, SOR ₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₅, COR₅, COOR₅,NR₆COR₅,SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₂ represents H, OH, Cl, Br, F, I, NO₂, CN, SO₂R₅, COR₅, COOR₅, NR₆COR₅, SOR₅, SR₅, PO₃R₆ R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C alkyloxy, C alkeny, C alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; T represents, CH₂=C, CH=CH, C(CH₃)=CH, C≡C, C=O, C=S, CONH, CSNH, COO, OCO, COS, COCH₂, or CH₂CO; A represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[12] A compound corresponding to general formula (7) 'Structure 7', 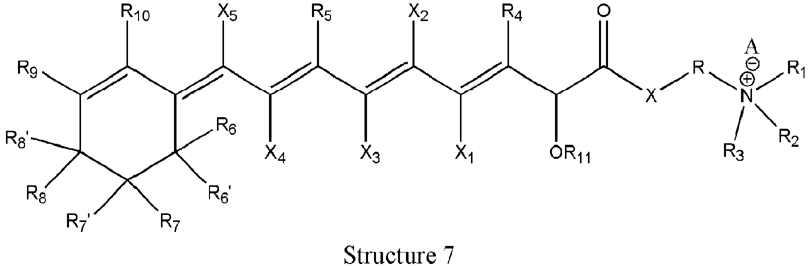 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR ₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR ₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ per-fluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆ COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR ₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃ R₇R₇', C₁₋₆alkyl, C₁₋₆alkyloxy, C₁₋₆alkeny, C₁₋₆alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇ and R₇' taken alone are same or different and are H, Cl ,Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₈ and R₈' taken alone are same or different and are H, Cl, Br, F, I, OH, NO₂, CN, SO₂R₅, COR₅, COOR₅, NR₆COR₅ , SOR₅, SR₅, PO₃R₆R₆', C₁₋₆ alkyl, C₁₋₆ alkyloxy, C ₁₋₆ alkeny, C ₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[13] A compound corresponding to general formula (8) 'Structure 8', 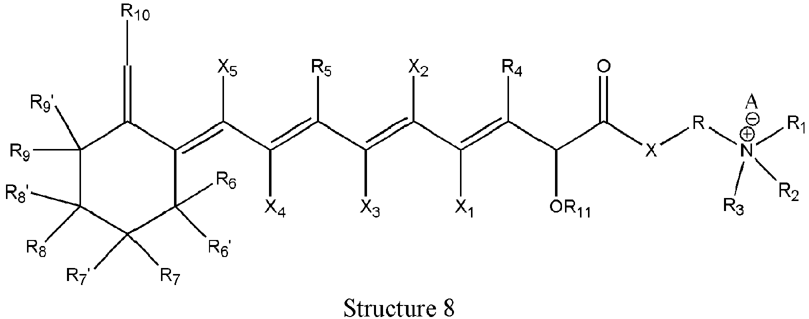 wherein, R represents a branched or straight chain, -(CH₂)ₙ-, wherein n=0, 1, 2, 3, 4, 5, 6, 7, 8, 9, 10 ..., in -(CH₂)ₙ- , any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO , CN, SO₂R₅, COR₅, COOR₅, NR ₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₅R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆ COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁-₆ alkeny, C ₁₋₆ alkyny, or C₁₋₆ alkyl halide; R₆ and R₆' taken alone are same or different and are H, Cl, Br, F, I, OH, NO , CN, SO₂R₅ , COR_{5,} COOR₅, NR₇COR₅, SOR₅, SR₅, PO₃R₇R₇', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇ and R₇' taken alone are same or different and are H, Cl, Br, F, I, OH, NO₂, CN, SO₂R₅ , COR_{5,} COOR₅, NR₆COR₅, SOR₅, SR₅, PO₃R₆R₆, C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₆R₆', aryl or heteroaryl residues, or other ring systems; R₈ and R₈' taken alone are same or different and are H, Cl, Br, F, I, OH, NO₂, CN, SO₂R₅ , COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₉ and R ' taken alone are same or different and are H, Cl, Br, F, I, OH, NO , CN, SO R , COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A⁻ represents Cl⁻, Br, F⁻, I⁻, AcO⁻, citrate, or any negative ions; the double bonds on the side chain may have Z or E configuration; All R, R₁, R₂, R₃, R₄, -(CH₂)ₙ-groups are branched or straight chains, and may contain C, H, O, S, N, and other atoms and may contain single, double, triple bonds and ring systems.
[14] A compound corresponding to general formula (9) 'Structure 9', 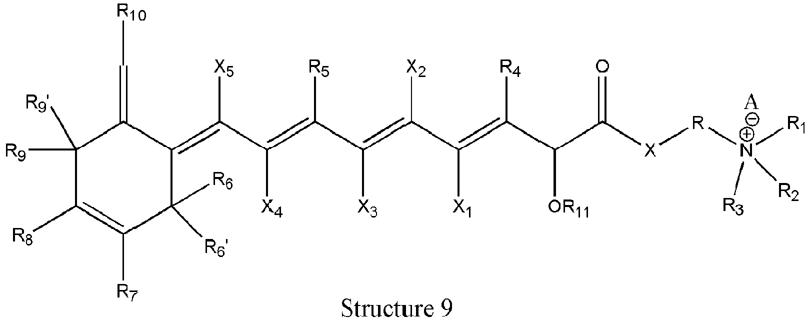 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆ COR₅, SOR₅, SR₅, PO₃ R₆ R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO_{2,} CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄, R₅, R₇, R₈, R₁₀, and R₁₁ are same or different and are H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₉COR₅ , SOR₅, SR₅, PO₃R₉R₉', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₉R₉', aryl or heteroaryl residues, or other ring systems; R₉ and R₉' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(H₂)ₙ-, wherein n=2, 3, 4, 5, 6, 7, 8, 9, 10,..., and any CH may be replaced with O, S, CH=CH, C≡C, CR₆R₆', aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[15] A compound corresponding to general formula (10) 'Structure 10', 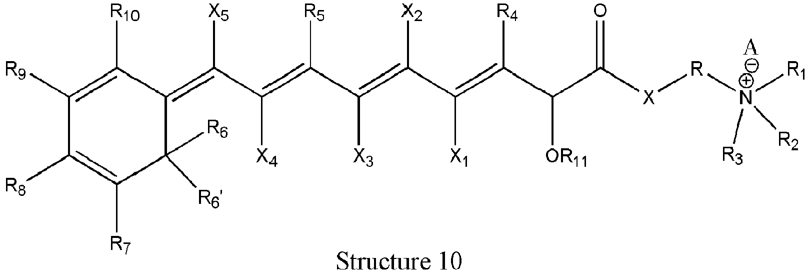 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂ , CN, SO₂R₅, COR₅, COOR₅, NR₆COR₅, SOR₅, SR₅ , PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆ COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄, R₅, R₇, R₈, R₉, C₁₀, and R₁₁ are same or different and are H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₁₂COR₅, SOR₅, SR₅, PO₃R₁₂R₁₂', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₉R₉', aryl or heteroaryl residues, or other ring systems; R₁₂ and R₁₂' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[16] A compound corresponding to general formula (11) 'Structure 11', 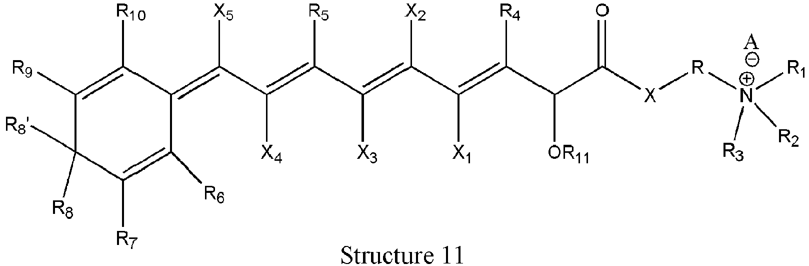 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆ COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆ COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂R₅, COR₅, COOR₅, NR₆COR₅, SOR₅, SR₅, PO₃R₆R₆', Calkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO_{2,} CN, SO₂R₅, COR₅, COOR₅, NR₆COR₅, SOR₅, SR₅, PO₃R₆R₆', C₁₋₆ alkyl, C₁₋₆ alkyloxy, C perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₅, COR₅, COOR₅, NR₆COR₅, SOR₅, SR₅, PO₃R₆ R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₈COR₅, SOR₅, SR₅, PO₃R₈R₈', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₈R₈', aryl or heteroaryl residues, or other ring systems; R₈ and R₈' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R ₁, R₂, R₃, R₄, -(CH₂)ₙ- groups are branched or straight chains, and may contain C, H, O, S, N, and other atoms and may contain single, double, triple bonds and ring systems.
[17] A compound corresponding to general formula (12) 'Structure 12', 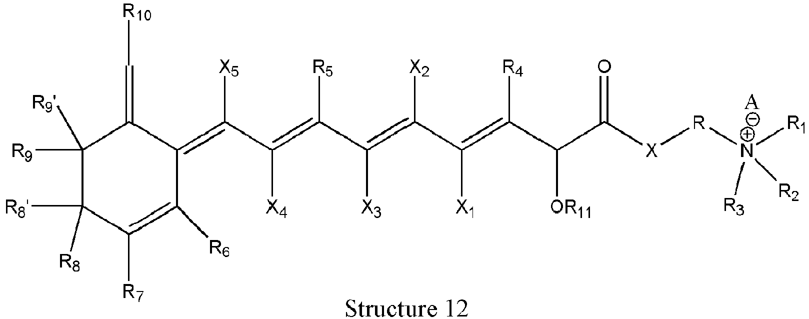 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆ COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO_{2,} CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆ COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO_{2,} CN, SO₂R₅, COR₂, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₅, COR₅, COOR₅, NR₆COR₅, SOR₅, SR₅, PO₃R₆ R₆ C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆ taken alone are same or different and are H, Cl, Br, F, I, OH, NO₂, CN, SO₂R₅, COR₅, COOR₅, NR₈COR₅, SOR₅, SR₅, PO₃R₈R₈',C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₈R₈', aryl or heteroaryl residues, or other ring systems; R₆ and R₈' taken alone are same or different and are H, Cl, Br, F, I, OH, NO₂,CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₆ and R₉' taken alone are same or different and are H, Cl, Br, F, I, OH, NO₂, CN, SO₂R₅, COR₅, COOR₅, NR₆COR₅, SOR₅ ,SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R ₁, R₂, R₃, R₄, -(CH₂)ₙ- groups are branched or straight chains, and may contain C, H, O, S, N, and other atoms and may contain single, double, triple bonds and ring systems.
[18] A compound corresponding to general formula (13) 'Structure 13', 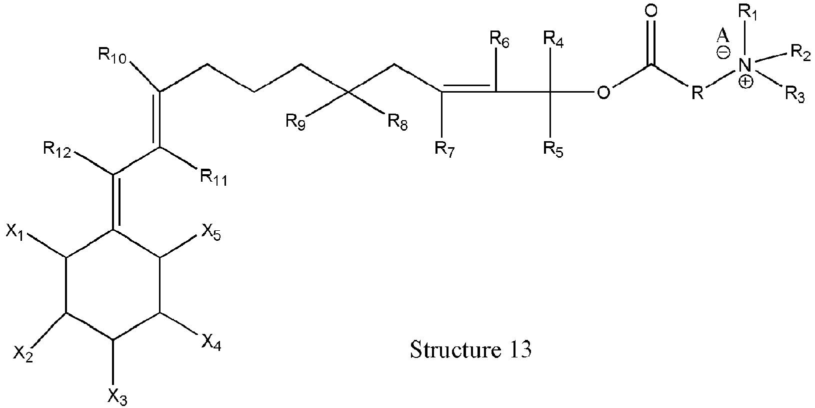 wherein, R represents a branched or straight chain, -(CH₂)ₙ-, wherein n=0, 1, 2, 3, 4, 5, 6, 7, 8, 9, 10 .... in -(CH₂)ₙ-, any CH₂ may be replaced with O, S, CH=CH, C≡C, CR₆R₇, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₇, aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₇, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₅R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ perfluoroalkyl, C ₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ per-fluoroalkyl, C ₁₋₆ alkeny, C ₁₋₆ alkyny, or C ₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆ COR₇, SOR₆, SR₆, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C ₁₋₆ alkyny, or C₁₋₆ alkyl halide; R₇ represents H, OH, Cl, Br, F, I, NO₂, CN, SO₂R₅, COR₅, COOR₅, NR₆COR₅, SOR ₅, SR_{5,} PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₈ represents H, OH, Cl, Br, F, I, NO₂, CN, SO ₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₇, C ₁₋₆ alkyl, C₁₋₆ alkyloxy, C₁₋₆ perfluoroalkyl, C₁₋₆ alkeny, C₁₋₆ alkyny, or C ₁₋₆ alkyl halide; R₁₀ represents H, OH, Cl, Br, F, I, NO₂, CN, SO₂R₅, COR₅, COOR₅, NR₆ COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₅, COR₅, COOR₅, NR₆COR₅, SOR₅, SR₅, PO₅R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₂ represents H, OH, Cl, Br, F, I, NO₂, CN, SO₂R₅, COR₅, COOR₅, NR₆COR₅, SOR ₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[19] A compound corresponding to general formula (14) 'Structure 14', 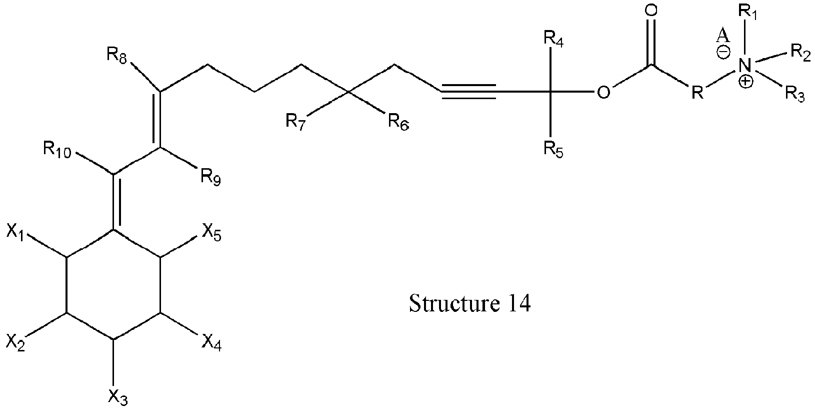 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₇, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₇, aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₇, C₁₋₆ alkyl, C₁₋₆ perfluoroalkyl, C₁₋₆ alkeny, C ₁₋₆ alkyny, or C₁₋₆ alkyloxy; X₂ represents H, OH, Cl, Br, F, I, NO₂, CN, SO₂R₅, COR₅, COOR₅, NR₆COR₅, SOR ₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR ₆COR₅, SOR₅, SR₅, PO₅R₆R₇, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₅R₆R₇, C₁₋₆ alkyl, C₁₋₆ perfluoroalkyl, C ₁₋₆ alkeny, C₁₋₆ alkyny, or C ₁₋₆ alkyloxy; X₅ represents H, OH, Cl, Br, F, I, NO₂, CN, SO₂R₅, COR₅, COOR₅, NR₆COR₅, SOR₅, SR₅, PO₅R₆R₇, C₁₋₆ alkyl, C₁₋₆ per-fluoroalkyl, C ₁₋₆ alkeny, C ₁₋₆ alkyny, or C ₁₋₆ alkyloxy; R ₄ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C ₁₋₆ alkeny, C ₁₋₆ alkyny, or C ₁₋₆ alkyl halide; R₅ represents H, OH, Cl, Br, F, I, NO₂, CN, SO₂R₆, COR₆, COOR₆, NR₆ COR₇, SOR₆, SR₆, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂R₅, COR₅, COOR₅, NR₆COR₅, SOR₅, SR₅, PO₅R₆R₇, C₁₋₆ alkyl, C₁₋₆ alkyloxy, C ₁₋₆ perfluoroalkyl, C ₁₋₆ alkeny, C ₁₋₆ alkyny, or C₁₋₆ alkyl halide; R₇ represents H, OH, Cl, Br, F, I, NO₂, CN, SO₂R₅, COR₅, COOR₅, NR₆COR₅, SOR ₅, SR₅, PO₅R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R ₈ represents H, OH, Cl, Br, F, I, NO₂, CN, SO₂R₅, COR₅, COOR₅, NR₆COR₅, SOR₅, SR₅, PO₅R₆R₇, C₁₋₆ alkyl, C₁₋₆ alkyloxy, C₁₋₆ perfluoroalkyl, C₁₋₆ alkeny, C₁₋₆ alkyny, or C ₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₇, C ₁₋₆ alkyl, C₁₋₆ alkyloxy, C₁₋₆ perfluoroalkyl, C₁₋₆ alkeny, C₁₋₆ alkyny, or C ₁₋₆ alkyl halide; R₁₀ represents H, OH, Cl, Br, F, I, NO₂, CN, SO₂R₅, COR₅, COOR₅, NR₆ COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[20] A compound corresponding to general formula (15) 'Structure 15', 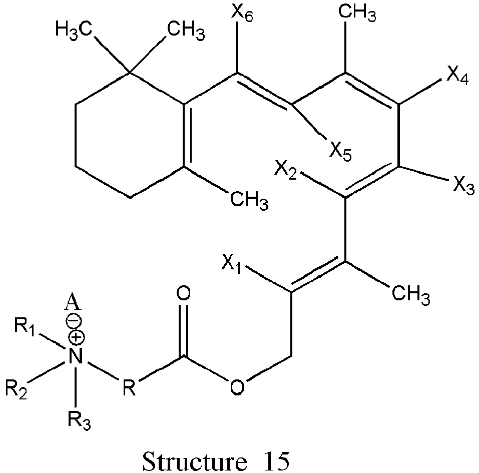 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₆', C₁₋₆ alkyl, C₁₋₆ perfluoroalkyl, C₁₋₆ alkeny, C ₁₋₆ alkyny, or C₁₋₆ alkyloxy; X₂ represents H, OH, Cl, Br, F, I, NO₂, CN, SO₂R₅, COR₅, COOR₅, NR₆COR₅, SOR ₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR ₆COR₅, SOR₅, SR₅, PO₃R₆R₆', C₁₋₆ alkyl, C₁₋₆ perfluoroalkyl, C₁₋₆ alkeny, C₁₋₆ alkyny, or C ₁₋₆ alkyloxy; X₄ represents H, OH, Cl, Br, F, I, NO₂, CN, SO₂R₅, COR₅, COOR₅, NR₆COR₅, SOR₅, SR₅, PO₅R₆R₆', C₁₋₆ alkyl, C₁₋₆ perfluoroalkyl, C₁₋₆ alkeny, C₁₋₆ alkyny, or C ₁₋₆ alkyloxy; X₅ represents H, OH, Cl, Br, F, I, NO₂, CN, SO₂R₅, COR₅, COOR₅, NR₆COR₅, SOR , SR₅, PO₃R₆R₆', C₁₋₆ alkyl, C₁₋₆ per-fluoroalkyl, C₁₋₆ alkeny, C₁₋₆ alkyny, or C₁₋₆ alkyloxy; X₆ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₆, COR₆, COOR₆, NR₆COR₆, SOR₆, SR₆, PO₃ R₆R₆', C₁₋₆ alkyl, C₁₋₆ alkyloxy, C₁₋₆ perfluoroalkyl, C₁₋₆ alkeny, C₁₋₆ alkyny, or ₁₋₆ alkyl halide; R₅ rpresents H, OH, Cl, Br, F, I, NO₂, CN, SO₂R₆, COR₆, COOR₆, NR₆COR₆, SOR₆, SR₆, PO₃R₆R₆', C₁₋₆ alkyl, C₁₋₆ alkyloxy, C₁₋₆ perfluoroalkyl, C ₁₋₆ alkeny, C₁₋₆ alkyny, or ₁₋₆ alkyl halide; R₆ represents H, OH, Cl, Br, F, I, NO₂, CN, SO₂R₅, COR₅, COOR₅, NR₅COR₄, SOR₅, SR₅, PO₅R₅R₄, C₁₋₆ alkyl, C₁₋₆ alkyloxy, C₁₋₆ perfluoroalkyl, C₁₋₆ alkeny, C₁₋₆ alkyny, or alkyl halide; R₆ and R ₆' taken alone are same or different and are H, Cl, Br, F, I, OH, NO₂, CN, SO₂R₅, COR₅, COOR₅, NR₄COR₅, SOR₅, SR₅, PO₅R₄R₅, C₁₋₆ alkyl, C₁₋₆ alkyloxy, C₁₋₆ alkeny, C₁₋₆ alkyny, or alkyl halide or taken together are oxygen (=O) or -(CH₂)ₙ - , wherein n=2, 3, 4, 5, 6, 7, 8, 9, 10,..., and any CH₂ may be replaced with O, S, CH=CH, C≡C, CR₄R₅, aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ-groups are branched or straight chains, and may contain C, H, O, S, N, and other atoms and may contain single, double, triple bonds and ring systems.
[21] A compound corresponding to general formula (16) 'Structure 16', 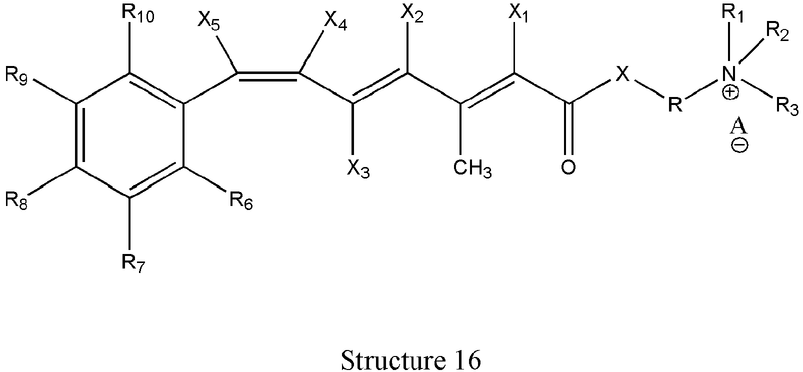 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₅, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₅, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₅, aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₅, C₁₋₆ alkyl, C₁₋₆ perfluoroalkyl, C ₁₋₆ alkeny, C ₁₋₆ alkyny, or C₁₋₆ alkyloxy; X₂ represents H, OH, Cl, Br, F, I, NO₂, CN, SO₂R₅, COR₅, COOR₅, NR₆COR₅, SOR ₅, SR₅, PO₃R₆R₅, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₅R₆R₅, C₁₋₆ alkyl, C₁₋₆ perfluoroalkyl, C_{1-6 1-6} alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₅, C₁₋₆ alkyl, C₁₋₆ per-fluoroalkyl, C₁₋₆ alkeny, C ₁₋₆ alkyny, or C ₁₋₆ alkyloxy; R ₄ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C ₁₋₆ alkeny, C ₁₋₆ alkyny, or C ₁₋₆ alkyl halide; R₅ represents H, OH, Cl, Br, F, I, NO₂, CN, SO₂R₆, COR₆, COOR₆, NR₆ COR₇, SOR₆, SR₆, PO₃R₆R₄, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂R₅, COR₅, COOR₅, NR₄COR₅, SOR₅, SR₅, PO₅R₄R₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂R₅, COR₅, COOR₅, NR₆COR₅, SOR ₅, SR₅, PO₃R₆R₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R ₈ represents H, OH, Cl, Br, F, I, NO₂, CN, SO₂R₅, COR₅, COOR₅, NR₄COR₅, SOR₅, SR₅, PO₅R₄R₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₇, C ₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆ COR₅, SOR₅, SR₅, PO₃R₆R₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[22] A compound corresponding to general formula (17) 'Structure 17', 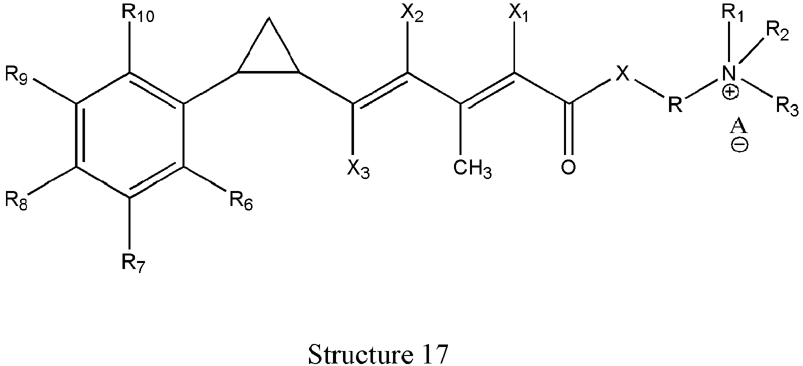 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₅, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₅, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₅, aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆ R₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR ₅, SR₅, PO₃R₆R₅, C₁₋₆ alkyl, C₁₋₆ perfluoroalkyl, C₁₋₆ alkenyl, C₁₋₆ alkyny, or C₁₋₆ alkyloxy; X₃ represents H, OH, Cl, Br, F, I, NO₂, CN, SO₂R₅, COR₅, COOR₅, NR ₆COR₅, SOR₅, SR₅, PO₅R₆R₅, SOR₅, SR₅, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₅, SOR₅, SR₅, C₁₋₆ alkyl, C ₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₇, SOR ₆, SR₆, PO₃R₆R₄,SOR₆, SR₆, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂R₅, COR₅, COOR₅, NR₄COR₅, SOR₅, SR₅, PO₃R₄R₅, SOR₅, SR₅, C₁₋₆ alkyl, C₁₋₆ alkyloxy, C₁₋₆ perfluoroalkyl, C₁₋₆ alkeny, C₁₋₆ alkyny, or C ₁₋₆ alkyl halide; R represents H, OH, Cl, Br, F, I, NO₂, CN, SO₂R₅, COR₅, COOR₅, NR₆ COR₅, SOR₅, SR₅, PO₃R₆R₅, SOR₅, SR₅, C₁₋₆ alkyl, C₁₋₆ alkyloxy, C₁₋₆ per-fluoroalkyl, C₁₋₆ alkeny, C₁₋₆ alkyny, or C ₁₋₆ alkyl halide; R ₈ represents H, OH, Cl, Br, F, I, NO₂, CN, SO₂R₅, COR₅, COOR₅, NR₄COR₅, SOR₅, SR₅, PO₃R₄R₅, SOR₅ , SR₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₅, SOR₅, SR₅, C₁₋₆ alkyl, C₁₋₆ alkyloxy, C₁₋₆ per-fluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR₅, SR₅, PO₃R₆R₅, SOR₅, SR₅, C₁₋₆ alkyl, C₁₋₆ alkyloxy, C₁₋₆ perfluoroalkyl, C₁₋₆ alkeny, C₁₋₆ alkyny, or C ₁₋₆ alkyl halide; A represents Cl⁻, Br⁻, F⁻, I⁻, AcO⁻, citrate, or any negative ions; the double bonds on the side chain may have Z or E configuration; All R, R ₁, R₂, R₃, R₄, -(CH₂)ₙ- groups are branched or straight chains, and may contain C, H, O, S, N, and other atoms and may contain single, double, triple bonds and ring systems.
[23] A compound corresponding to general formula (18) 'Structure 18', 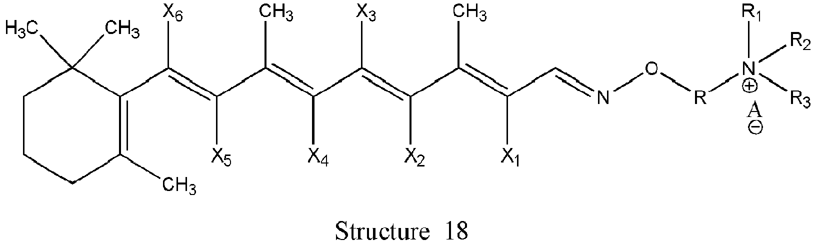 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₅, COX₅, COOX₅, NX₆COX₅, SOX₅, SX₅, PO₃X₆ X₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₅, COX₅, COOX₅, NX₆ COX₅, SOX₅, SX₅, PO₃X₆X₅, C₁₋₆ alkyl, C₁₋₆ perfluoroalkyl, C₁₋₆ alkeny, C ₁₋₆ alkyny, or C₁₋₆ alkyloxy; X₅ represents H, OH, Cl, Br, F, I, NO₂, CN, SO₂X₄, COX₄, COOX₄, NX₆COX₄, SOX₄, SX₄, PO₃C₆C₄, C₁₋₆ alkyl, C₁₋₆ per-fluoroalkyl, C₁₋₆ alkeny, C₁₋₆ alkyny, or C₁₋₆ alkyloxy; X₆ represents H, OH, Cl, Br, F, I, NO₂, CN, SO₂X₅, COX₅, COOX₅, NX₄COX₅, SOX₅, SX₅, PO₃X₄X₅, C₁₋₆ alkyl, C₁₋₆ perfluoroalkyl, C₁₋₆ alkeny, C₁₋₆ alkyny, or C₁₋₆ alkyloxy; A⁻ represents Cl⁻, Br⁻, F⁻, I⁻, AcO⁻, citrate, or any negative ions; the double bonds on the side chain may have Z or E configuration; All R, R , R₂, R₃, R₄, -(CH₂)ₙ - groups are branched or straight chains, and may contain C, H, O, S, N, and other atoms and may contain single, double, triple bonds and ring systems.
[24] A compound corresponding to general formula (19) 'Structure 19', 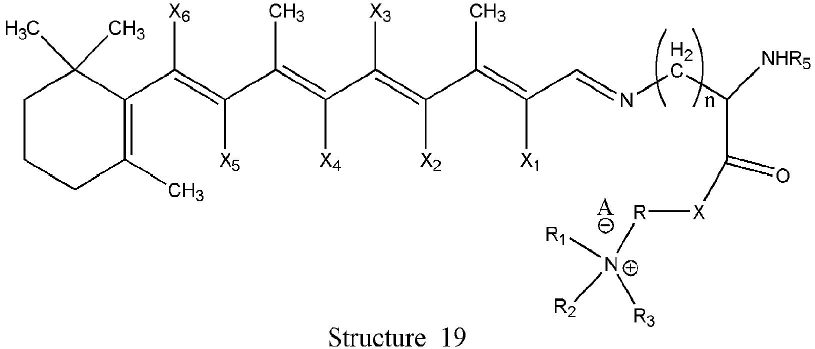 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₅, COX₅, COOX₅, NX₆COX₅, SOX₅, SX₅, PO₃X₆ X₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy,; X₄ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₆COX₄, SOX₄, SX₄, PO₃X₆X₄, C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂X₅, COX₅, COOX₅, NX₄COX₅, SOX₅, SX₅, PO₃X₄X₅, C₁₋₆ alkyl, C₁₋₆ perfluoroalkyl, C₁₋₆ alkenyl, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃ R₅X₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₄COX₅, SOR₄, SR₄, PO₃R₄X₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[25] A compound corresponding to general formula (20) 'Structure 20', 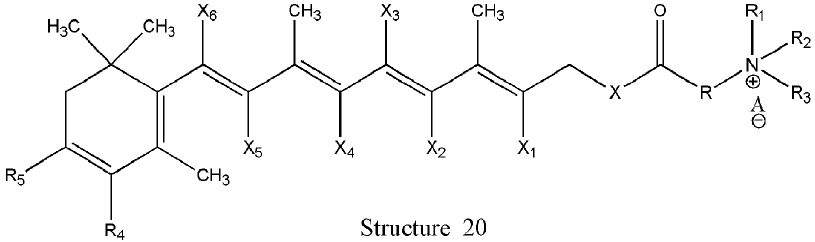 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₅, COX₅, COOX₅, NX₆COX₅, SOX₅, SX₅, PO₃X₆ X₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₅, COX₅, COOX₅, NX₆COX₅, SOX₅, SX₅, PO₃X₆X₅, C alkyl, C perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₆COX₄, SOX₄, SX₄, PO₃X₆X₄, C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂X₅, COX₅, COOX₅, NX₄COX₅, SOX₅, SX₅, PO₃X₄X₅, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO , CN, SO₂X₅, COX₅, COOX₅, NX₆COX₅, SOX₅, SX₅, PO ₃X₄X₅ C₁₋₆ alkyl, C₁₋₆ alkyloxy, C₁₋₆ perfluoroalkyl, C₁₋₆ alkeny, C₁₋₆ alkyny, or C ₁₋₆ alkyl halide; R₅ represents H, OH, Cl, Br, F, I, NO₂, CN, SO₂X₄, COX₄, COOX₄, NX₄COX₅, SOX₅, SX₄, PO₃X₄X₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[26] A compound corresponding to general formula (21) 'Structure 21', 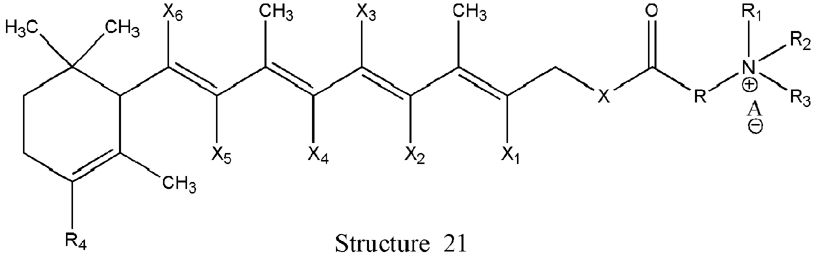 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₅, COX₅, COOX₅, NX₆COX₅, SOX₅, SX₅, PO₃X₆ X₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₆COX₄, SOX₄, SX₄, PO₃X₆X₄, C₁₋₆ alkyl, C₁₋₆ perfluoroalkyl, C₁₋₆ alkeny, Calkyny, or Calkyloxy; X₆ represents H, OH, Cl, Br, F, I, NO₂, CN, SO₂X₅, COX₅, COOX₅, NX₄COX₅, SOX₅, SX₅, PO₃X₄X₅, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃ R₅X₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₄COX₁, SOR₄, SR₄, PO₃R₄X₁, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R , R , R , R , -(CH₂)ₙ- groups are branched or straight chains, 1 2 3 4 2 n and may contain C, H, O, S, N, and other atoms and may contain single, double, triple bonds and ring systems.
[27] A compound corresponding to general formula (22) 'Structure 22', 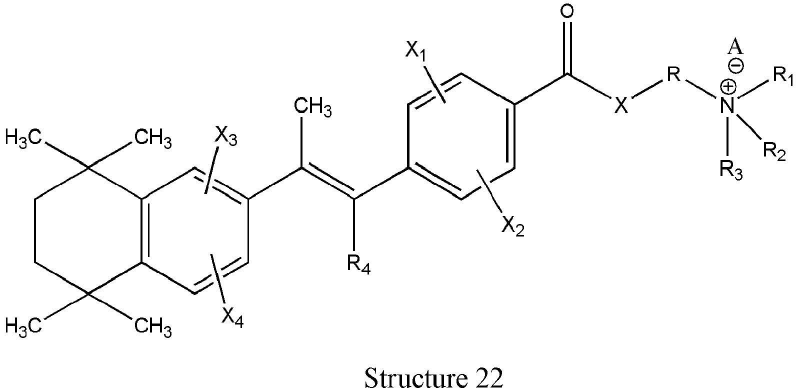 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂ X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₄COX₁, SOR ₄, SR₄, PO₃R₄X₁, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[28] A compound corresponding to general formula (23) 'Structure 23', 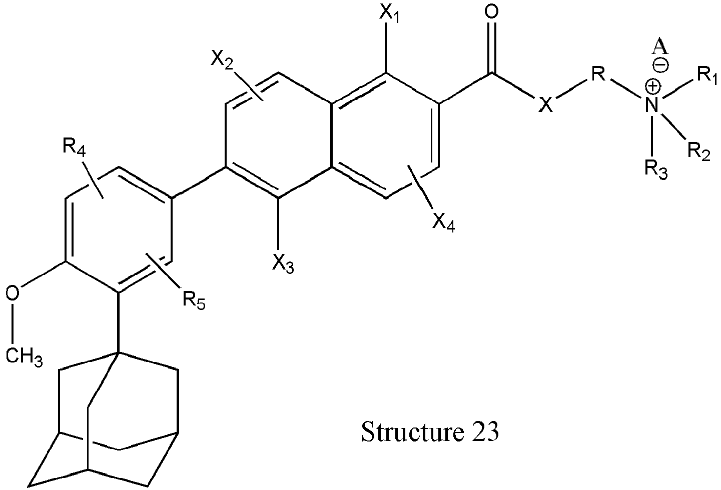 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂ X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₄COX₁, SOR ₄, SR₄, PO₃R₄X₁, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[29] A compound corresponding to general formula (24) 'Structure 24', 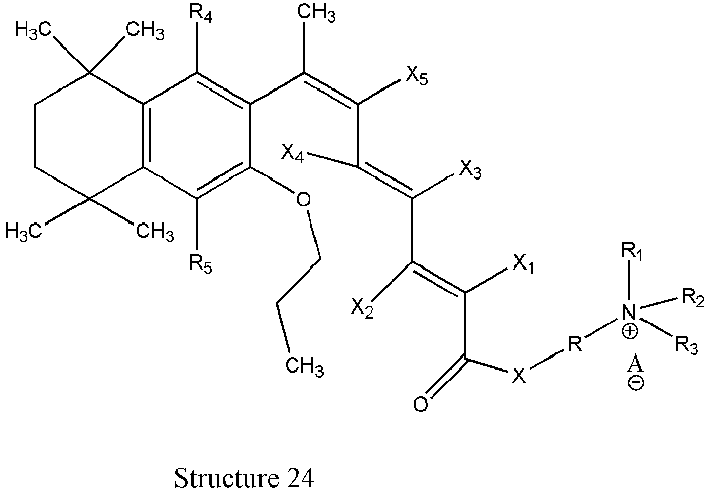 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂ X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₁COX₄, SOX₄, SX₄, PO₃X₁X₄, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R represents H, OH, Cl, Br, F, I, NO₂, CN, SO₂R₄, COR₄, COOR₄, NR₄ COX₅, SOR₄, SR₄, PO₃R₄X₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions. The double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.
[30] A compound corresponding to general formula (25) 'Structure 25', 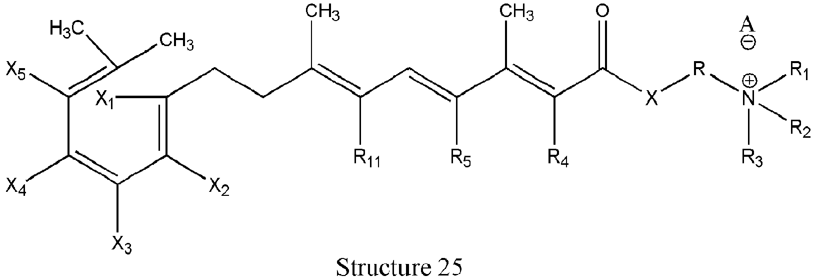 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C=C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂ X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₁COX₄, SOX₄, SX₄, PO₃X₁X₄, C alkyl, C perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁ COX₂, SOX₁, SX₁, PO₃X₁X₂, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R represents H, OH, Cl, Br, F, I, NO₂, CN, SO₂R₄, COR₄, COOR₄, NR₄ COX₁, SOR₄, SR₄, PO₃R₄X₁, C₁₋₆ alkyl, C₁₋₆ alkyloxy, C₁₋₆ perfluoroalkyl, C₁₋₆ alkeny, C₁₋₆alkyny, or C₁₋₆ alkyl halide; R₁₁ represents H, OH, Cl, Br, F, I, NO₂, CN, SO₂R₄, COR₄, COOR , NR₄COX₁, SOR₄, SR , PO₃R₄X₁, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ-groups are branched or straight chains, and may contain C, H, O, S, N, and other atoms and may contain single, double, triple bonds and ring systems.
[31] A compound corresponding to general formula (26) 'Structure 26', 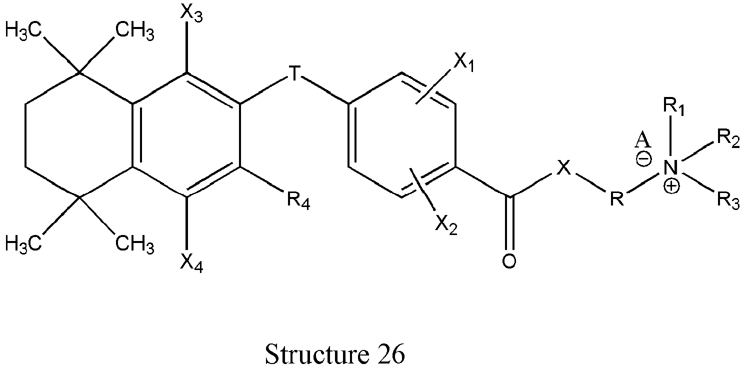 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂ COX₃, SOX₂, SX₃, PO₃X₂ X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁ COX₃, SOX₁, SX₁, PO₃ X₁ X₃, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁ COX₂, SOX₁, SX₁, PO₃X₁X₂, C₁₋₆ alkyl, C₁₋₆ perfluoroalkyl, C₁₋₆ alkeny, C₁₋₆alkyny, or C₁₋₆ alkyloxy; X₅ represents H, OH, Cl, Br, F, I, NO₂, CN, SO₂X₄, COX₄, COOX₄, NX₁COX₄, SOX₄, SX₄, PO₃X₁X₄, C alkyl, C₁₋₆ per-fluoroalkyl, C₁₋₆ alkeny, C₁₋₆alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₁₆ alkyl, C ₁₋₆ alkyloxy, C₁₋₆ perfluoroalkyl, C₁₋₆ alkeny, C₁₋₆alkyny, or C₁₋₆ alkyl halide; T represents, CH₂=C, CH=CH, C(CH₃)=CH, C≡C, C=O, C=S, CONH, CSNH, COO, OCO, COS, COCH₂, or CH₂CO; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight 1 2 3 4 2 n chains, and may contain C, H, O, S, N, and other atoms and may contain single, double, triple bonds and ring systems.
[32] A compound corresponding to general formula (27) 'Structure 27', 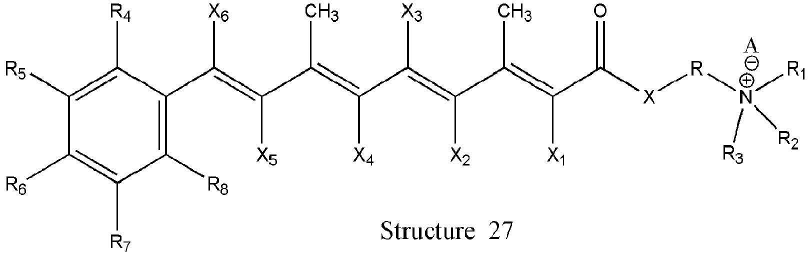 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂ X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO , CN, SO₂ X₁, COX₁, COOX₁, NX₁ COX₃, SOX₁, SX₁, PO₃X₁X₃, C₁₋₆ alkyl, C₁₋₆ perfluoroalkyl, C₁₋₆ alkeny, C₁₋₆ alkyny, or C ₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆alkyny, or C₁₋₆ alkyloxy; X₅ represents H, OH, Cl, Br, F, I, NO₂, CN, SO₂X₄, COX₄, COOX₄, NX₁COX₄, SOX₄, SX₄, PO₃X₁X₄, C₁₋₆ alkyl, C₁₋₆ per-fluoroalkyl, C₁₋₆ alkeny, C ₁₋₆alkyny, or C₁₋₆ alkyloxy; X₅ represents H, OH, Cl, Br, F, I, NO₂, CN, SO₂X₄, COX₄, COOX₄, NX₁ COX₄, SOX₄, SX₄, PO₃X₁X₄, C₁₋₆ alkyl, C₁₋₆ perfluoroalkyl, C₁₋₆ alkeny, C₁₋₆alkyny, or C₁₋₆ alkyloxy; R₄ represents H, OH, Cl, Br, F, I, NO , CN, SO₂X₁, COX , COOX₁, NX₁COX₂, SOX₁, SX₁, PO ₃X₁X₂, C₁₋₆ alkyl, C₁₋₆ alkyloxy, C₁₋₆ perfluoroalkyl, C₁₋₆ alkeny, C₁₋₆ alkyny, or C ₁₋₆ alkyl halide; R₅ represents H, OH, Cl, Br, F, I, NO₂, CN, SO₂ X₁, COX₁, COOX₁, NX₁ COX₂, SOX₁, SX₁, PO₃X₁X₂, C₁₋₆ alkyl, C₁₋₆ alkyloxy, C₁₋₆ per-fluoroalkyl, C₁₋₆ alkeny, C₁₋₆ alkyny, or C₁₋₆ alkyl halide; R₆ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂X₁, COX₁, COOX₁, NX₁ COX₂, SOX₁, SX₁, PO₃X₁X₂, C₁₋₆ alkyl, C₁₋₆ alkyloxy, C₁₋₆ perfluoroalkyl, C₁₋₆ alkeny, C₁₋₆alkyny, or C₁₋₆ alkyl halide; R₈ represents H, OH, Cl, Br, F, I, NO₂, CN, SOX₁, COX₁, COOX₁, NX₁COX₂, SOX₁, SX₁, PO₃X₁X₂, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions. The double bonds on the side chain may have Z or E configuration. All R, R₁, R₂, R₃, R₄, -(CH₂)ₙ-groups are branched or straight chains, and may contain C, H, O, S, N, and other atoms and may contain single, double, triple bonds and ring systems.
[33] Processes for the preparation of compounds of the general formula (1, 2, 3, 5, 6, 7, 8, 9, 10, 11, 12, 16, 17, 22, 23, 24, 25, 26, and 27) 'Structure 1, 2, 3, 5, 6, 7, 8, 9, 10, 11, 12, 16, 17, 22, 23, 24, 25, 26, and 27' according to 1, 2, 3, 4, 5, 6, 9, 10, 11, 12, 13, 14, 15, 16, 17, 21, 22, 27, 28, 29, 30, 31, and 32, wherein the compounds can be prepared from retinoic acids and related compounds, by reaction with compounds of the general formula (28) 'Structure 28' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Ben-zotriazol-1-yl-oxy-tris(dimethylamino)phosphonium hexafluorophosphate, et al., 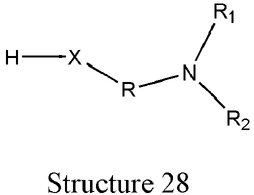 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆ R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆ R₆', aryl or heteroaryl residues, or other ring systems; R₄ represents H, OH, Cl, Br, F, I, NO₂, CN, SO₂R₅, COR₅, COOR₅, NR₆COR₅, SOR₅, SR₅, PO₃ R₆R₆', C₁₋₆ alkyl, C₁₋₆ alkyloxy, C₁₋₆ perfluoroalkyl, C ₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₆COR₄, SOR₄, SR , PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅ , COR₅, COOR₅, NR₄COR₅, SOR₅, SR₅, PO₃R₅R₄, C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₄R₅, aryl or heteroaryl residues, or other ring systems; X represents O, S, or NH. The double bonds on the side chain may have Z or E configuration. All R, R₁, R₂, R₃, R₄, -(CH₂)ₙ- groups are branched or straight chains, and may contain C, H, O, S, N, and other atoms and may contain single, double, triple bonds and ring systems.
[34] Processes for the preparation of compounds of the general formula (4 , 13, 14, 15, 20, and 21) 'Structure 4 , 13, 14, 15, 20, or 21' according to 7, 8, 18, 19, 20, 23, 24, 25, and 26, wherein the compounds can be prepared from retinol and related compounds, by reaction with compounds of the general formula (29) 'Structure 29', 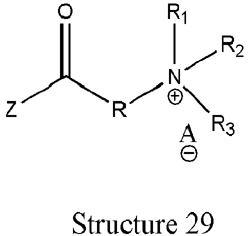 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R and R taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; R₄ represents H, OH, Cl, Br, F, I, NO₂, CN, SO₂R ₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆alkyny, or C₁₋₆ alkyl halide; R₅ represents H, OH, Cl, Br, F, I, NO₂, CN, SO₂R₄, COR₄, COOR₄, NR₆COR₄, SOR₄, SR₄, PO₃R₆R₆', C ₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₄COR₅, SOR₅, SR₅, PO₃R₅R₄, C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₄R₅, aryl or heteroaryl residues, or other ring systems; Z represents F, Cl, Br, or I; A⁻ represents Cl⁻, Br⁻, F⁻, I⁻, AcO⁻, citrate, or any negative ions. The double bonds on the side chain may have Z or E configuration. All R, R₁, R₂, R₃, R₄, -(CH₂)ₙ- groups are branched or straight chains, and may contain C, H, O, S, N, and other atoms and may contain single, double, triple bonds and ring systems.
[35] Processes for the preparation of compounds of the general formula (1, 2, 3, 5, 6, 7, 8, 9, 10, 11, 12, 16, 17, 22, 23, 24, 25, 26, and 27) 'Structure 1, 2, 3, 5, 6, 7, 8, 9, 10, 11, 12, 16, 17, 22, 23, 24, 25, 26, and 27' according to 1, 2, 3, 4, 5, 6, 9, 10, 11, 12, 13, 14, 15, 16, 17, 21, 22, 27, 28, 29, 30, 31, and 32, wherein the compounds can be prepared from metal salts, organic base salts , or immobilized base salts of retinoic acids or related compounds when X represents O, by reaction with compounds of the general formula (30) 'Structure 30', 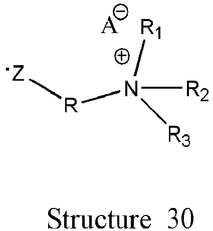 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R and R taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; R₄ represents H, OH, Cl, Br, F, I, NO₂, CN, SO₂R ₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆alkyny, or C₁₋₆ alkyl halide; R₅ represents H, OH, Cl, Br, F, I, NO₂, CN, SO₂R₄, COR₄, COOR₄, NR₆COR₄, SOR₄, SR₄, PO₃R₆R₆', C ₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO , CN, SO₂R₅, COR₅, COOR₅, NR₄COR₅, SOR₅, SR₅, PO₃R₅R₄, C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₄R₅, aryl or heteroaryl residues, or other ring systems; Z represents halogen, or p-toluenesulphonyl; A⁻ represents Cl⁻, Br⁻, F⁻, I⁻, AcO⁻, citrate, or any negative ions. The double bonds on the side chain may have Z or E configuration. All R, R₁, R₂, R₃, R₄, -(CH₂)ₙ- groups are branched or straight chains, and may contain C, H, O, S, N, and other atoms and may contain single, double, triple bonds and ring systems.
[36] Compounds of the general formulas (1 to 27) 'Structure 1 to 27' or a composition comprising of at least one compound of the general formulas (1 to 27)' Structure 1 to 27', as an active ingredient, according to 1 to 32, can be administered orally or transdermally, for treating any retinoids and retinoid-like compounds-treatable conditions in humans or animals. The retinoids and retinoid-like compounds-treatable conditions include, but are not limited to acne, acne scarring, psoriasis, ichthyosis, eczema, keratinization disorders, precancerous lesions, chemoprophylaxis, warts, sarcoidosis, treating photoaged skin, preventing photoaged skin, treating chronologically aged skin, hair loss, and various cancers etc..
[37] Methods for treating any retinoids and retinoid-like compounds-treatable conditions in humans or animals by administering transdermally (topically) to any part of body (in the form of a solution, spray, lotion, ointment, emulsion or gel) to deliver therapeutically effective plasma levels of the compounds of the general formulas (1 to 27)' Structure 1 to 27', as an active ingredient, according to 1 to 32.
[38] Methods for topically treating acne, acne scarring, psoriasis, ichthyosis, eczema, keratinization disorders, precancerous lesions, chemoprophylaxis, warts, sarcoidosis, treating photoaged skin, preventing photoaged skin, treating chronologically aged skin, hair loss, and various cancers etc. in humans or animals by administering to the particular site of disease a therapeutically effective amount of the compounds of the general formulas (1 to 27)' Structure 1 to 27', as an active ingredient, according to 1 to 32.
[39] Transdermal therapeutic application systems of compounds of general formulas (1 to 27)' Structure 1 to 27', as an active ingredient, according to 1 to 32, for treating any retinoids and retinoid-like compounds-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables the retinoids and retinoid-like compounds to reach constantly optimal therapeutic blood levels to increase effectiveness and reduce the side effects of retinoids and retinoid-like compounds.

## Claims

1. A compound corresponding to the general formula (2) 'Structure 2', 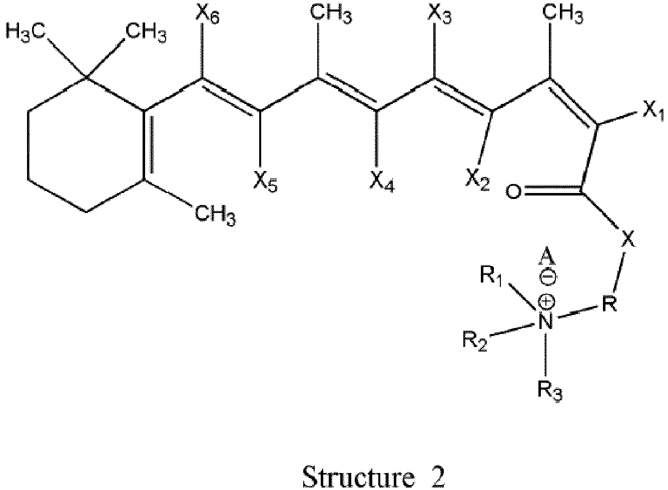 wherein, R represents a branched or straight chain, -(C₂H)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆ COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₅ represents H, OH, Cl, Br, F, I, NO₂, CN, SO₂R₆, COR₆, COOR₆, NR₆COR₇, SOR₆, SR₆, PO₃R₆R₆', C₁₋₆ alkyl, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C₁₋₆ alkyloxy, C₁₋₆ alkeny, C₁₋₆ alkyny, or alkyl halide or taken together are oxygen (=O) or -(CH₂)ₙ -, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or-CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A⁻ represents Cl⁻, Br, F, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; optionally wherein the compound is N,N-diethylaminoethyl 13-cis-retinoate.HA, wherein, A⁻ represents Cl⁻, Br, F, I⁻, AcO⁻, citrate, or any negative ions.

2. A compound corresponding to the general formula (3) 'Structure 3', 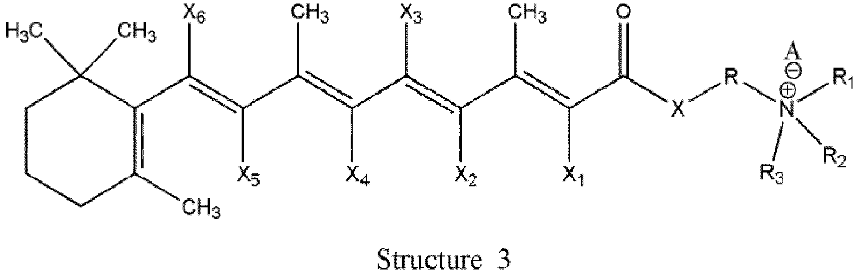 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₅ represents H, OH, Cl, Br, F, I, NO₂, CN, SO₂R₆, COR₆, COOR₆, NR₆COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C₁₋₆ alkyloxy, C₁₋₆ alkyloxy, C₁₋₆ alkeny, C₁₋₆ alkyny or alkyl halide or taken together are oxygen (=O) or-(CH₂)ₙ -, wherein n=2, 3, 4, 5, 6, 7, 8, 9, 10,..., and any CH₂ may be replaced with O, S, CH=CH, C≡C, CR₇R₇', aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ -, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A represents Cl, Br, F, I, AcO, citrate, or any negative ions; the double bonds on the side chain may have Z or E configuration; All R, R₁, R₂, R₃, R₄, -(CH₂)ₙ - groups are branched or straight chains, and may contain C, H, O, S, N, and other atoms and may contain single, double, triple bonds and ring systems; optionally wherein the compound is N,N-diethylaminoethyl all- trans-retinoate.HA, wherein, A represents Cl⁻, Br, F, I⁻, AcO⁻, citrate, or any negative ions.

3. A compound corresponding to general formula (4) 'Structure 4', 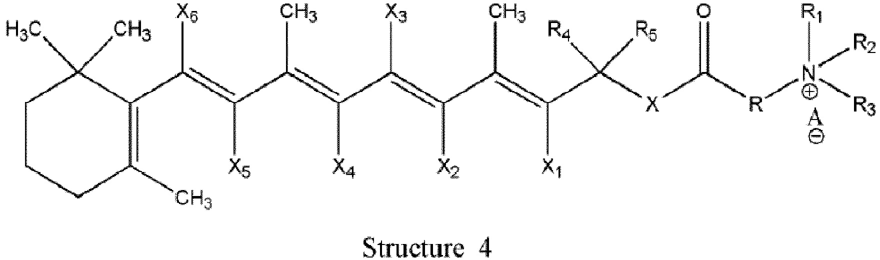 wherein, R represents a branched or straight chain, -(CH₂)ₙ -, wherein n=0, 1, 2, 3, 4, 5, 6, 7, 8, 9, 10 ..., in -(CH₂)ₙ -, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ -, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇^{'}, aryl or heteroaryl residues, or other ring systems; R₇ and R₇^{'} taken alone are same or different and are H, Cl, Br, F, I, OH, NO₂, CN, SO₂ R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ -, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; A- represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; optionally wherein the compound is retinyl N,N-dimethyl-2-aminoacetate.HA, wherein, A⁻represents Cl⁻, Br⁻, F⁻, I⁻, AcO⁻, citrate, or any negative ions.

4. A compound corresponding to the general formula (5) 'Structure 5', 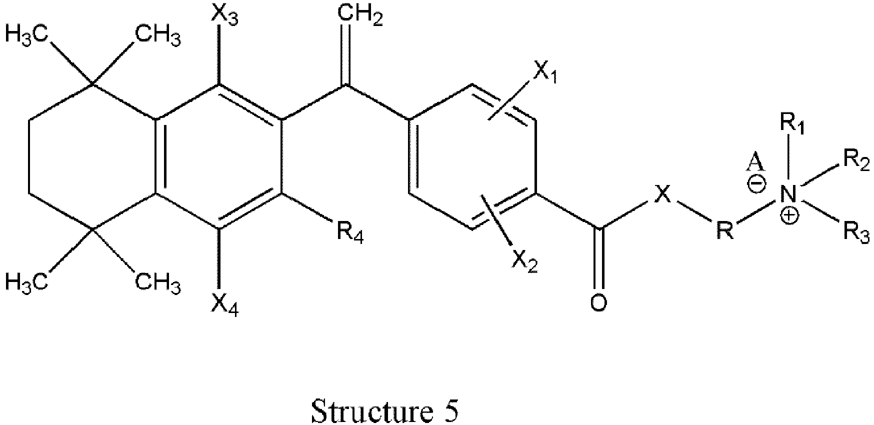 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ can be replaced with O, S, CH=CH, C≡C, CR₆R₆', aryl or heteroaryl residues, or other ring systems; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇, COR₅, SOR₅, SR₅,' PO₃R₇R₇', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇^{'}, aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems ; optionally wherein the compound is N,N-diethylaminoethyl 4-[1-(5,6,7,8-tetrahydro-3,5,5,8,8-pentamethyl-2-naphthalenyl)ethenyl]benzoate. H A, wherein, A⁻ represents Cl⁻, Br⁻, F⁻, I⁻, AcO⁻, citrate, or any negative ions.

5. A compound corresponding to general formula (6) 'Structure 6', 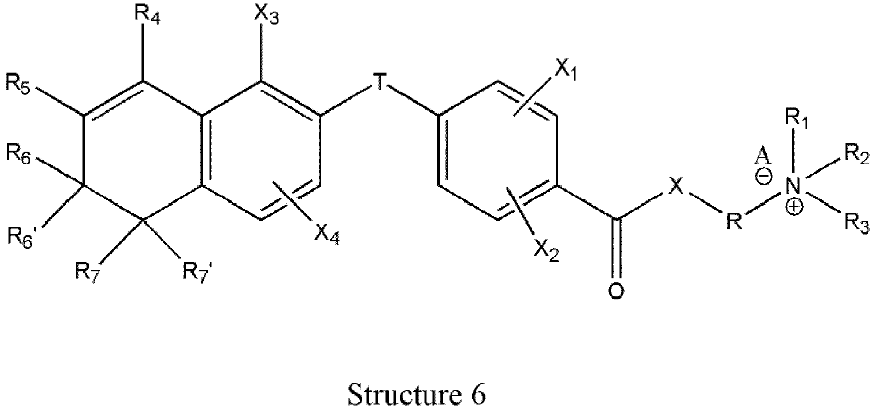 wherein R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C=C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₇, SOR₆, SR₆, PO₃R₆R₆', C₁₋₆ alky, C₁₋₆ alkyloxy, C₁₋₆ perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C₁₋₆ alkyloxy, C₁₋₆ alkeny, C₁₋₆ alkyny, or alkyl halide or taken together are oxygen (=O) or -(CH₂)ₙ -, wherein n=2, 3, 4, 5, 6, 7, 8, 9, 10,..., and any CH₂ may be replaced with O, S, CH=CH, C≡C, CR₇R₇^{'}, aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ -, wherein n=2, 3, 4, 5, 6, 7, 8, 9, 10,..., and any CH₂ may be replaced with O, S, CH=CH, C=C, CR₆R₆', aryl or heteroaryl residues, or other ring systems; T represents, CH₂ =C, CH=CH, C(CH₃)=CH, C≡C, C=O, C=S, CONH, CSNH, COO, OCO, COS, COCH₂, or CH₂CO; A represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems ; or a compound corresponding to general formula (7) 'Structure 7', 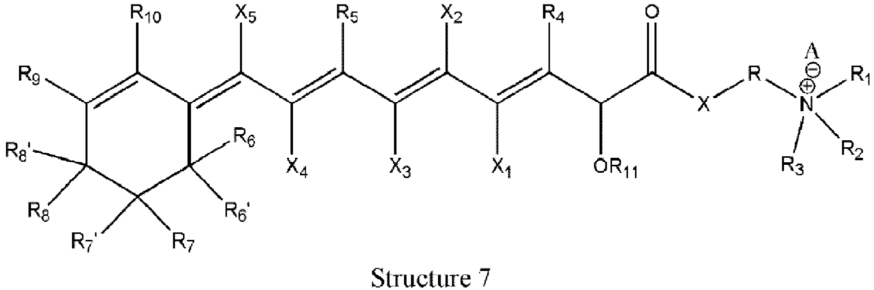 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆ COR₇, SOR₆, SR₆, PO₃R₆R₆', C₁₋₆ alky, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃R₇R₇', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₇R₇^{'}, aryl or heteroaryl residues, or other ring systems; R₇ and R₇' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₈ and R₈' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems ; or a compound corresponding to general formula (8) 'Structure 8', 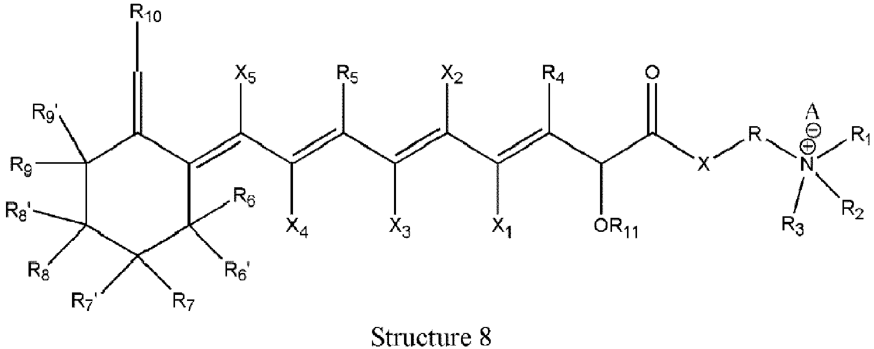 wherein, R represents a branched or straight chain, -(CH₂)ₙ-, wherein n=0, 1, 2, 3, 4, 5, 6, 7, 8, 9, 10 ..., in -(CH₂)ₙ -, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₇COR₅, SOR₅, SR₅, PO₃,R₇R₇', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₇R₇^{'}, aryl or heteroaryl residues, or other rings systems; R₇ and R₇' taken alone are same or different and are H, Cl, Br, F, I, OH, NO₂, CN, SO₂R₅, COR₅, COOR₅, NR₆COR₅, SOR₅, SR₅, PO₃,R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₆R₆', aryl or heteroaryl residues, or other rings systems; R₈ and R₈' taken alone are same or different and are H, Cl, Br, F, I, OH, NO₂, CN, SO₂R₅, COR₅, COOR₅, NR₆COR₅, SOR₅, SR₅, PO₃,R₆R₆', C₁₋₆ alkyl, C₁₋₆ alkyloxy, C₁₋₆ alkeny, C₁₋₆ alkyny, or alkyl halide or taken together are oxygen (=O) or-(CH₂)ₙ-, wherein n=2, 3, 4, 5, 6, 7, 8, 9, 10,..., and any CH₂ may be replaced with O, S, CH=CH, C=C, CR₆R₆', aryl or heteroaryl residues, or other rings systems; R₉ and R₉' taken alone are same or different and are H, Cl, Br, F, I, OH, NO₂, CN, SO₂R₅, COR₅, COOR₅, NR₇COR₅, SOR₅, SR₅, SR₅, PO₃,R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₆R₆', aryl or heteroaryl residues, or other rings systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a compound corresponding to general formula (9) 'Structure 9', 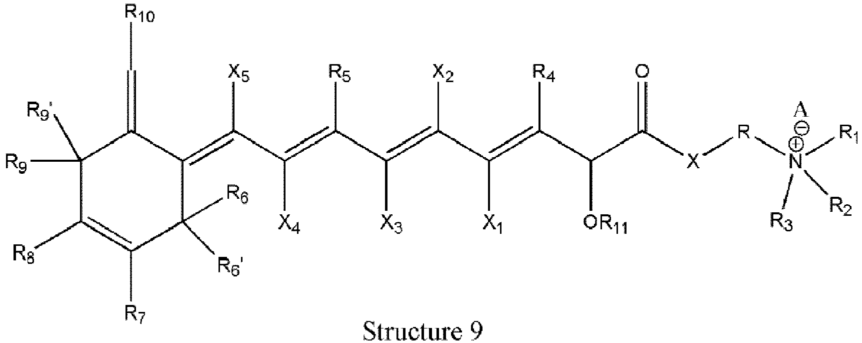 wherein, R represents a branched or straight chain, -(CH₂)ₙ-, wherein n=0, 1, 2, 3, 4, 5, 6, 7, 8, 9, 10 ..., in -(CH₂)ₙ -, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄, R₅, R₇, R₈, R₁₀, and R₁₁ are same or different and are H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₉COR₅, SOR₅, SR₅, PO₃,R₉R₉', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₉R₉', aryl or heteroaryl residues, or other rings systems; R₉ and R₉' taken alone are same or different and are H, Cl, Br, F, I, OH, NO₂, CN, SO₂R₅, COR₅, COOR₅, NR₆COR₅, SOR₅, SR₅, PO₃,R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₆R₆', aryl or heteroaryl residues, or other rings systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems or a compound corresponding to general formula (10) 'Structure 10', 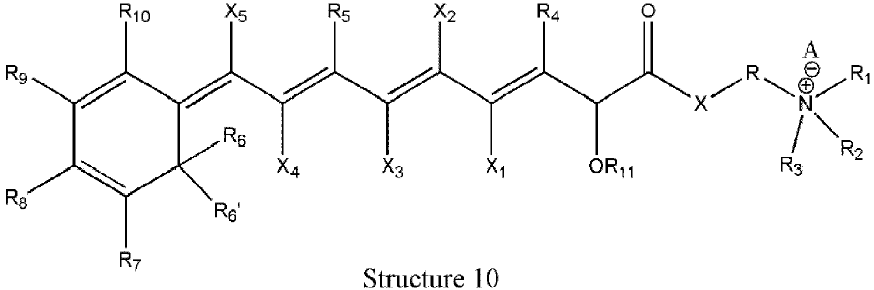 wherein, R represents a branched or straight chain, -(CH₂)ₙ-, wherein n=0, 1, 2, 3, 4, 5, 6, 7, 8, 9, 10 ..., in -(CH₂)ₙ -, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄, R₅, R₇, R₈, R₉, R₁₀, and R₁₁ are same or different and are H, Cl, Br, F, I, OH, NO₂, CN, SO₂R₅, COR₅, COOR₅, NR₁₂COR₅, SOR₅, SR₅, PO₃R₁₂R₁₂', C₁₋₆ alkyl, C₁₋₆ alkyloxy, C₁₋₆ perfluoroalkyl, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₉R₉', aryl or heteroaryl residues, or other rings systems; R₁₂ and R₁₂' taken alone are same or different and are H, Cl, Br, F, I, OH, NO₂, CN, SO₂R₅, COR₅, COOR₅, NR₆COR₅, SOR₅, SR₅, PO₃,R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₆R₆', aryl or heteroaryl residues, or other rings systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a compound corresponding to general formula (11) 'Structure 11', 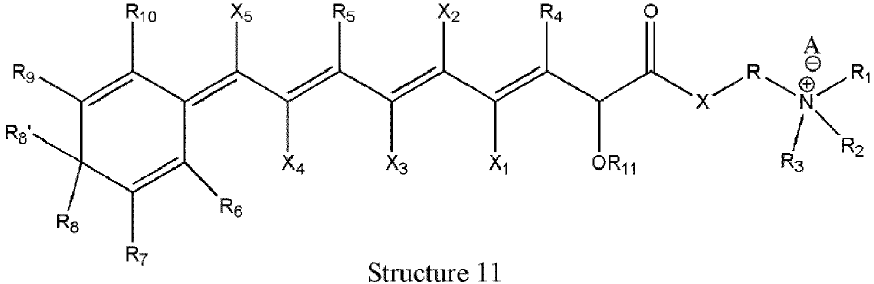 wherein, R represents a branched or straight chain, -(CH₂)ₙ-, wherein n=0, 1, 2, 3, 4, 5, 6, 7, 8, 9, 10 ..., in -(CH₂)ₙ -, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₅, COR₅, COOR₅, NR₆COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₈COR₅, SOR₅, SR₅, PO₃,R₈R₈', C₁₋₆ alkyl, C₁₋₆ alkyloxy, C₁₋₆ perfluoroalkyl, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₈R₈', aryl or heteroaryl residues, or other rings systems; R₈ and R₈'taken alone are same or different and are H, Cl, Br, F, I, OH, NO₂, CN, SO₂R₅, COR₅, COOR₅, NR₆COR₅, SOR₅, SR₅, PO₃,R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₆R₆', aryl or heteroaryl residues, or other rings systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.

6. A compound corresponding to general formula (12) 'Structure 12', 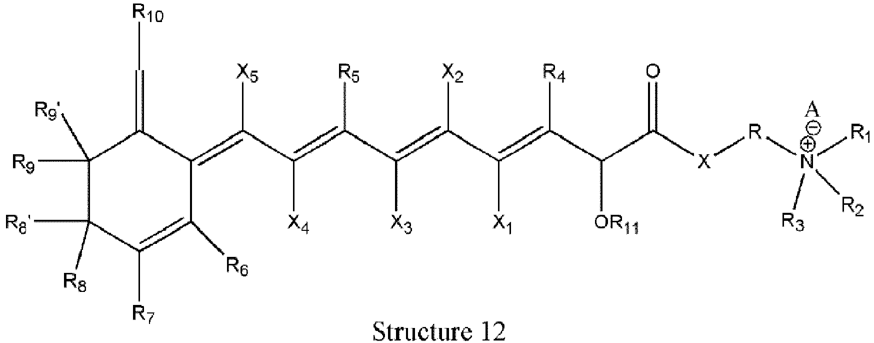 wherein, R represents a branched or straight chain, -(CH₂)ₙ-, wherein n=0, 1, 2, 3, 4, 5, 6, 7, 8, 9, 10 ..., in -(CH₂)ₙ -, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, NO, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₇, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₈COR₅, SOR₅, SR₅, PO₃,R₈R₈', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C=C, CR₈R₈', aryl or heteroaryl residues, or other ring systems; R₈ and R₈' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or - (CH₂)ₙ-, wherein n=2, 3, 4, 5, 6, 7, 8, 9, 10,..., and any CH may be replaced with O, S, CH=CH, C≡C, CR₆R₆', aryl or heteroaryl residues, or other ring systems; R₉ and R₉' taken alone are same or different and are H, Cl, Br, F, I, OH, NO₂, CN, SO₂R₅, COR₅, COOR₅, NR₆COR₅, SOR₅, SR₅, PO₃R₆R₆', C₁₋₆ alkyl, C₁₋₆ alkyloxy, C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH may be replaced with O, S, CH=CH, C=C, CR₆R₆', aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ-groups are branched or straight chains, and may contain C, H, O, S, N, and other atoms and may contain single, double, triple bonds and ring systems; or a compound corresponding to general formula (13) 'Structure 13', 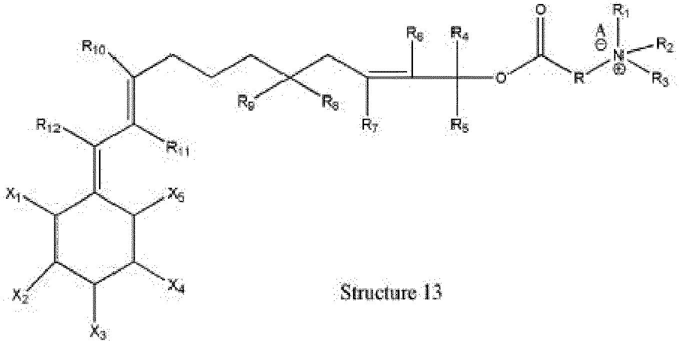 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₇, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₇, aryl or heteroaryl residues, or other ring systems; X represents O, S, or NH; X₁ represents H, OH, Cl, Br, F, I, NO₂, NO, CN, SO₂R₅, COR₅, COOR₅, NR₆ COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₇, SOR₆, SR₆, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₈ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₂ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ - groups are branched or straight chains, and may contain C, H, O, S, N, and other atoms and may contain single, double, triple bonds and ring systems; or a compound corresponding to general formula (14) 'Structure 14', 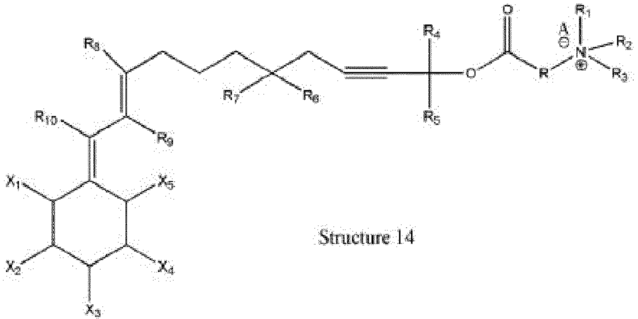 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₇, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₇, aryl or heteroaryl residues, or other ring systems; X represents O, S, or NH; X₁ represents H, OH, Cl, Br, F, I, NO₂, NO, CN, SO₂R₅, COR₅, COOR₅, NR₆ COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆COR₇, SOR₆, SR₆, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂R₅, COR₅, COOR₅, NR₆ COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂R₅, COR₅, COOR₅, NR₆ COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₈ represents H, OH, Cl, Br, F, I, NO₂, CN, SO₂R₅, COR₅, COOR₅, NR₆ COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂ R₅, COR₅, COOR₅, NR₆COR₅, SOR₅, SR₅, PO₃R₆R₇, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ - groups are branched or straight chains, and may contain C, H, O, S, N, and other atoms and may contain single, double, triple bonds and ring systems; or a compound corresponding to general formula (15) 'Structure 15', 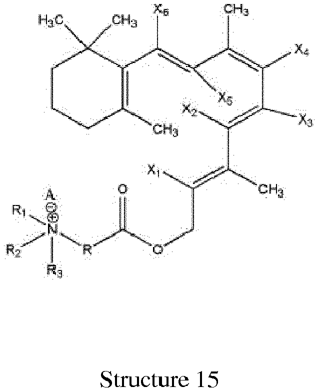 wherein, R represents a branched or straight chain, -(CH₂)ₙ-, wherein n=0, 1, 2, 3, 4, 5, 6, 7, 8, 9, 10 ..., in -(CH ₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R6', C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆ COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂ R₅, COR₅, COOR₅, NR₆ COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂ R₅, COR₅, COOR₅, NR₆COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂R₅, COR₅, COOR₅, N₆ COR₅, SOR₅, SR₅, PO₃R₆R₆', C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₆, COR₆, COOR₆, NR₆ COR_{6'}, SOR₆, SR₆, PO₃ R₆R₆', C₁₋₆ alkyl, C₁₋₆ alkyloxy, C₁₋₆ perfluoroalkyl, C₁₋₆ alkeny, C₁₋₆ alkyny, or ₁₋₆ alkyl halide; R₅ rpresents H, OH, Cl, Br, F, I, NO₂, CN, SO₂R₆, COR₆, COOR₆, NR₆ COR₆, SOR₆, SR₆, PO₃R₆R₆', C₁₋₆ alkyl, C₁₋₆ alkyloxy, C₁₋₆ perfluoroalkyl, C₁₋₆ alkeny, C₁₋₆ alkyny, or ₁₋₆ alkyl halide; R₆ represents H, OH, Cl, Br, F, I, NO₂, CN, SO₂R₅, COR₅, COOR₅, NR₅ COR₄, SOR₅, SR₅, PO₃R₅R₄, C₁₋₆ alkyl, C₁₋₆ alkyloxy, C₁₋₆ perfluoroalkyl, C₁₋₆ alkeny, C₁₋₆ alkyny, or ₁₋₆ alkyl halide; R₆ and R₆' taken alone are same or different and are H, Cl, Br, F, I, OH, NO₂, CN, SO₂R₅, COR₅, COOR₅, NR₄ COR₅, SOR₅, SR₅, PO₃R₄R₅, C₁₋₆ alkyl, C₁₋₆ alkyloxy, C₁₋₆alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₄R₅, aryl or heteroaryl residues, or other ring systems; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ - groups are branched or straight chains, and may contain C, H, O, S, N, and other atoms and may contain single, double, triple bonds and ring systems; or a compound corresponding to general formula (16) 'Structure 16', 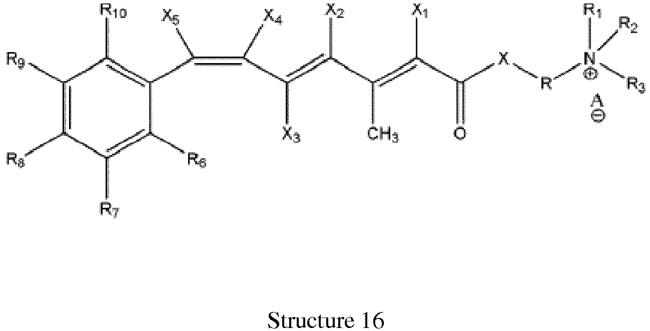 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₅, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₅, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₅, aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₅, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₅, C₁₋₆ alkyl, C₁₋₆ perfluoroalkyl, C₁₋₆ alkeny, C₁₋₆alkyny, or C₁₋₆ alkyloxy; X₄ represents H, OH, Cl, Br, F, I, NO₂, CN, SO₂ R₅, COR₅, COOR₅, NR₆ COR₅, SOR₅, SR₅, PO₃R₆R₅, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂ R₅, COR₅, COOR₅, NR₆COR₅, SOR₅, SR₅, PO₃R₆R₅, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂R₅, COR₅, COOR₅, NR₆ COR₅, SOR₅, SR₅, PO₃R₆R₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₆, COR₆, COOR₆, NR₆ COR₇, SOR₆, SR₆, PO₃R₆R₄, C₁₋₆ alkyl, C₁₋₆ alkyloxy, C₁₋₆ perfluoroalkyl, C₁₋₆ alkeny, C₁₋₆ alkyny, or ₁₋₆ alkyl halide; R₆ represents H, OH, Cl, Br, F, I, NO₂, CN, SO₂R₅, COR₅, COOR₅, NR₄COR₅, SOR₅, SR₅, PO₃R₄R₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂R₅, COR₅, COOR₅, NR₆COR₅, SOR₅, SR₅, PO₃R₆R₅, C₁₋₆ alkyl, C₁₋₆alkyloxy, C₁₋₆ perfluoroalkyl, C₁₋₆ alkeny, C₁₋₆ alkyny, or C₁₋₆ alkyl halide; R₈ represents H, OH, Cl, Br, F, I, NO₂, CN, SO₂R₅, COR₅, COOR₅, NR₄COR₅, SOR₅, SR₅, PO₃R₄R₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR₅, SR₅, PO₃R₆R₅, C₁₋₆ alkyl, C₁₋₆alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ - groups are branched or straight chains, and may contain C, H, O, S, N, and other atoms and may contain single, double, triple bonds and ring systems; or a compound corresponding to general formula (17) 'Structure 17', 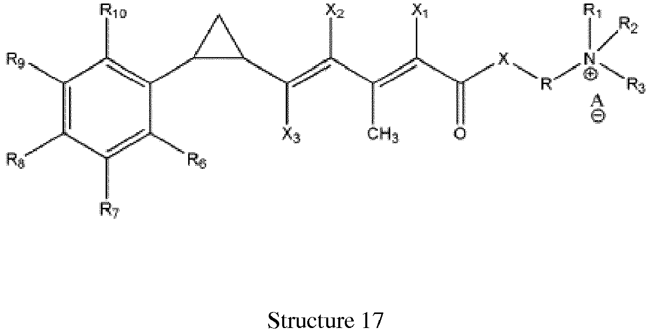 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₅, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₅, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₅, aryl or heteroaryl residues, or other ring systems; X represents O, S, or NH; X₁ represents H, OH, Cl, Br, F, I, NO₂, NO, CN, SO₂R₅, COR₅, COOR₅, NR₆COR₅, SOR₅, SR₅, PO₃R₆R₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂R₅, COR₅, COOR₅, NR₆COR₅, SOR₅, SR₅, PO₃R₆R₅, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂R₅, COR₅, COOR₅, NR₆COR₅, SOR₅, SR₅, PO₃R₆R₅, SOR₅, SR₅, C₁₋₆ alkyl, C₁₋₆ perfluoroalkyl, C₁₋₆ alkeny, C₁₋₆alkyny, or C₁₋₆ alkyloxy; R₄ represents H, OH, Cl, Br, F, I, NO₂, CN, SO₂ R₅, COR₅, COOR₅, NR₆ COR₅, SOR₅, SR₅, PO₃R₆R₅, SOR₅, SR₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂ R₆, COR₆, COOR₆, NR₆COR₇, SOR₆, SR₆, PO₃R₆R₄, SOR₆, SR₆, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂R₅, COR₅, COOR₅, NR₄ COR₅, SOR₅, SR₅, PO₃R₄R₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂R₅, COR₅, COOR₅, NR₆ COR₅, SOR₅, SR₅, PO₃R₆R₅, SOR₅, SR₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₈ represents H, OH, Cl, Br, F, I, NO₂, CN, SO₂R₅, COR₅, COOR₅, NR₄COR₅, SOR₅, SR₅, PO₃R₄R₅, SOR₅, SR₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₉ represents H, OH, Cl, Br, F, I, NO₂, CN, SO₂R₅, COR₅, COOR₅, NR₆COR₅, SOR₅, SR₅, PO₃R₆R₅, SOR₅, SR₅, C₁₋₆ alkyl, C₁₋₆alkyloxy, C₁₋₆ perfluoroalkyl, C₁₋₆ alkeny, C₁₋₆ alkyny, or C₁₋₆ alkyl halide; R₁₀ represents H, OH, Cl, Br, F, I, NO₂, CN, SO₂R₅, COR₅, COOR₅, NR₆COR₅, SOR₅, SR₅, PO₃R₆R₅, SOR₅, SR₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ - groups are branched or straight chains, and may contain C, H, O, S, N, and other atoms and may contain single, double, triple bonds and ring systems.

7. A compound corresponding to general formula (18) 'Structure 18', 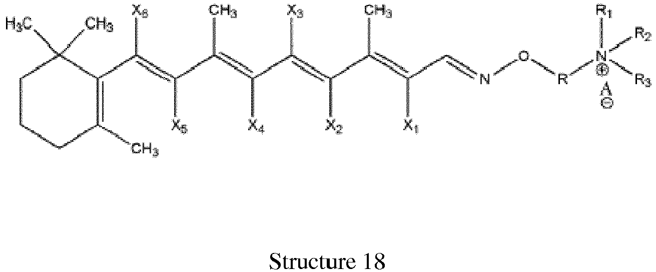 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₆COX₄, SOX₄, SX₄, PO₃X₆X₄, C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂X₅, COX₅, COOX₅, NX₄COX₅, SOX₅, SX₅, PO₃X₄X₅, C₁₋₆ alkyl, C₁₋₆ perfluoroalkyl, C₁₋₆ alkeny, C₁₋₆ alkyny, or C₁₋₆ alkyloxy; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a compound corresponding to general formula (19) 'Structure 19', 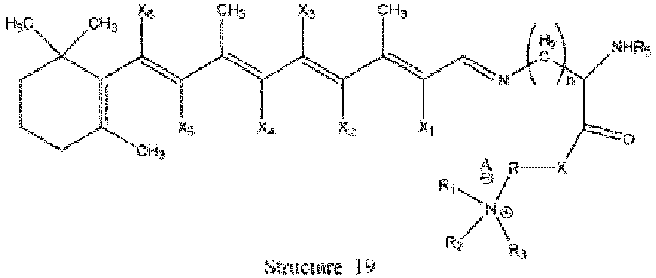 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₆COX₄, SOX₄, SX₄, PO₃X₆X₄, C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂X₅, COX₅, COOX₅, NX₄COX₅, SOX₅, SX₅, PO₃X₄X₅, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂ R₅, COR₅, COOR₅, NR₆ COR₅, SOR₅, SR₅, PO₃R₅X₅, C₁₋₆ alkyl, C₁₋₆ alkyloxy , C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂ R₄, COR₄, COOR₄, NR₄ COX₅, SOR₄, SR₄, PO₃R₄X₅, C₁₋₆ alkyl, C₁₋₆ alkyloxy , C₁₋₆ perfluoroalkyl, C₁₋₆ alkeny, C₁₋₆ alkyny, or C₁₋₆ alkyl halide; A⁻ represents Cl⁻, Br, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a compound corresponding to general formula (20) 'Structure 20', 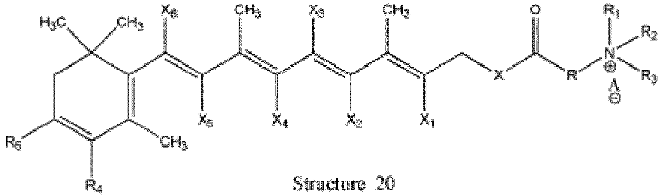 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂ , NO, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₆COX₄, SOX₄, SX₄, PO₃X₆X₄, C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂X₅, COX₅, COOX₅, NX₄COX₅, SOX₅, SX₅, PO₃X₄X₅, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂ X₅, COX₅, COOX₅, NX₆COX₅, SOX₅, SX₅, PO₃X₄X₅, C₁₋₆ alkyl, C₁₋₆ alkyloxy , C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂ X₄, COX₄, COOX₄, NX₄ COX₅, SOX₄, SX₄, PO₃X₄X₅, C₁₋₆ alkyl, C₁₋₆ alkyloxy , C₁₋₆ perfluoroalkyl, C₁₋₆ alkeny, C₁₋₆ alkyny, or C₁₋₆ alkyl halide; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a compound corresponding to general formula (21) 'Structure 21', 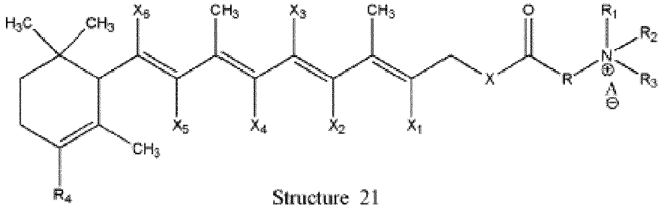 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₅, COX₅, COOX₅, NX₆COX₅, SOX₅, SX₅, PO₃X₆X₅, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₆COX₄, SOX₄, SX₄, PO₃X₆X₄, C₁₋₆ alkyl, C₁₋₆ perfluoroalkyl, C₁₋₆ alkeny, C₁₋₆ alkyny, or C₁₋₆ alkyloxy; X₆ represents H, OH, Cl, Br, F, I, NO₂, CN, SO₂X₅, COX₅, COOX₅, NX₄COX₅, SOX₅, SX₅, PO₃X₄X₅, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂ R₅, COR₅, COOR₅, NR₆COR₅, SOR₅, SR₅, PO₃R₅X₅, C₁₋₆ alkyl, C₁₋₆ alkyloxy , C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂ R₄, COR₄, COOR₄, NR₄ COX₁, SOR₄, SR₄, PO₃R₄X₁, C₁₋₆ alkyl, C₁₋₆ alkyloxy , C₁₋₆ perfluoroalkyl, C₁₋₆ alkeny, C₁₋₆ alkyny, or C₁₋₆ alkyl halide; A⁻ represents Cl⁻, Br, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a compound corresponding to general formula (22) 'Structure 22', 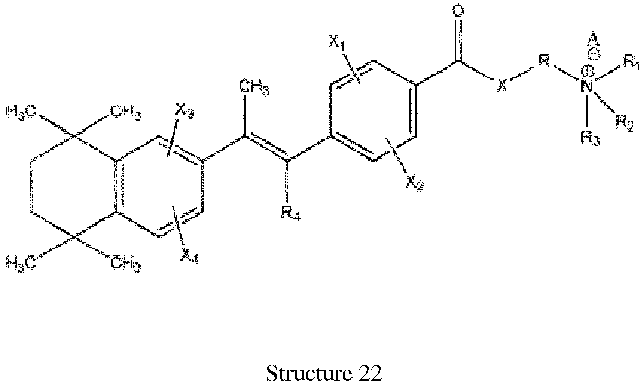 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₄COX₁, SOR₄, SR₄, PO₃R₄X₁, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.

8. A compound corresponding to general formula (23) 'Structure 23', 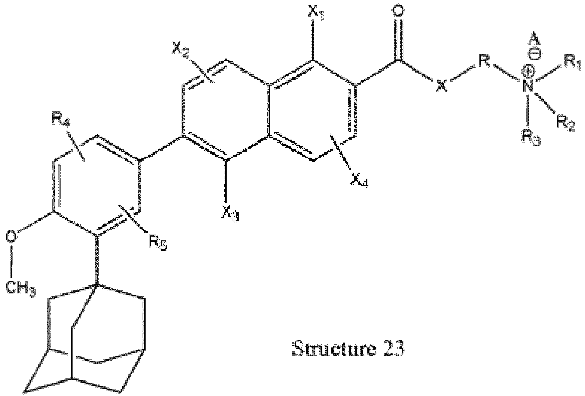 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₄COX₁, SOR₄, SR₄, PO₃R₄X₁, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A represents Cl⁻, Br, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a compound corresponding to general formula (24) 'Structure 24', 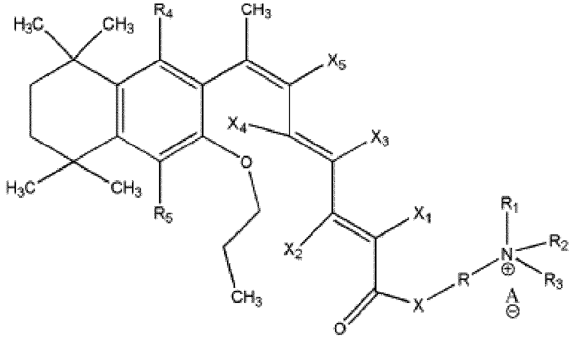 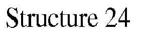 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₁COX₄, SOX₄, SX₄, PO₃X₁X₄, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₄COX₅, SOR₄, SR₄, PO₃R₄X₅, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br, F⁻, I⁻, AcO⁻, citrate, or any negative ions. The double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a compound corresponding to general formula (25) 'Structure 25', 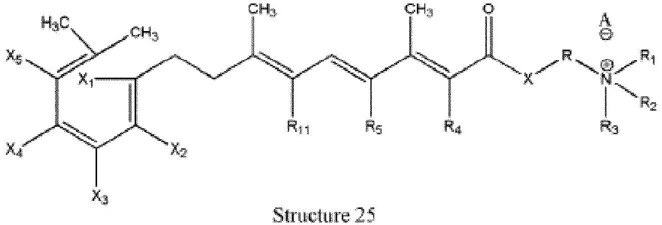 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₁COX₄, SOX₄, SX₄, PO₃X₁X₄, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₄COX₁, SOR₄, SR₄, PO₃R₄X₁, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₁₁ represents H, OH, Cl, Br, F, I, NO₂, CN, SO₂R₄, COR₄, COOR₄, NR₄COX₁, SOR₄, SR₄, PO₃R₄X₁, C₁₋₆ alkyl, C₁₋₆ alkyloxy, C₁₋₆ perfluoroalkyl, C₁₋₆ alkeny, C₁₋₆ alkyny, or C₁₋₆ alkyl halide; A⁻ represents Cl⁻, Br, F, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a A compound corresponding to general formula (26) 'Structure 26', 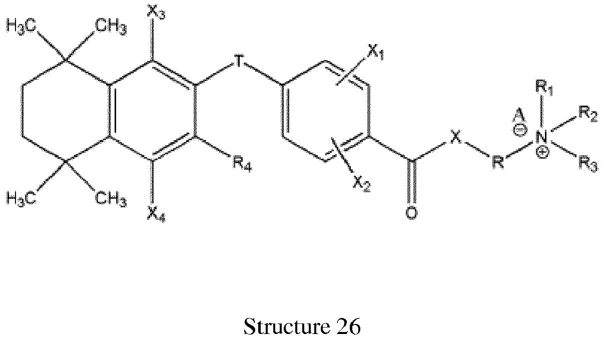 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₁COX₄, SOX₄, SX₄, PO₃X₁X₄, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy; C₁₋₆ perfluoroalkyl, C₁₋₆ alkeny, C₁₋₆ alkyny, or C₁₋₆ alkyl halide; T represents CH₂=C, CH=CH, C(CH₃)=CH, C≡C, C=O, C=S, CONH, CSNH, COO, OCO, COS, COCH₂, or CH₂CO; A⁻ represents Cl⁻, Br⁻, F⁻, I⁻, AcO⁻, citrate, or any negative ions; the double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems; or a compound corresponding to general formula (27) 'Structure 27', 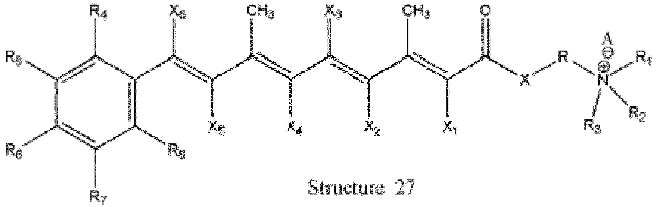 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CX₁X₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CX₁X₂, aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CX₁X₂, aryl or heteroaryl residues, or other ring systems; X represents O, S, or NH; X₁ represents H, OH, Cl, Br, F, I, NO₂, NO, CN, SO₂X₂, COX₂, COOX₅, NX₂COX₃, SOX₂, SX₃, PO₃X₂X₃, C₁₋₆ alkyl, C₁₋₆ perfluoroalkyl, C₁₋₆ alkeny, C₁₋₆ alkyny, or C₁₋₆ alkyloxy; X₂ represents H, OH, Cl, Br, F, I, NO₂, CN, SO₂X₁, COX₁, COOX₁, NX₁COX₃, SOX₁, SX₁, PO₃X₁X₃, C₁₋₆ alkyl, C₁₋₆ perfluoroalkyl, C₁₋₆ alkeny, C₁₋₆ alkyny, or C₁₋₆ alkyloxy; X₃ represents H, OH, Cl, Br, F, I, NO₂, CN, SO₂X₁, COX₁, COOX₁, NX₁COX₁, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ perfluoroalkyl, C₁₋₆ alkeny, C₁₋₆ alkyny, or C₁₋₆ alkyloxy; X₅ represents H, OH, Cl, Br, F, I, NO₂, CN, SO₂X₄, COX₄, COOX₄, NX₁COX₄, SOX₄, SX₄, PO₃X₁X₄, C₁₋₆ alkyl, C₁₋₆ perfluoroalkyl, C₁₋₆ alkeny, C₁₋₆ alkyny, or C₁₋₆ alkyloxy; R₄ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₇ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₈ represents H, OH, Cl, Br, F, I, NO₂, CN, SO₂X₁, COX₁, COOX₁, NX₁COX₂, SOX₁, SX₁, PO₃X₁X₂, C₁₋₆ alkyl, C₁₋₆ alkyloxy; C₁₋₆ perfluoroalkyl, C₁₋₆ alkeny, C₁₋₆ alkyny, or C₁₋₆ alkyl halide;
A⁻ represents Cl⁻, Br⁻, F⁻, I⁻, AcO⁻, citrate, or any negative ions. The double bonds on the side chain may have Z or E configuration; All R, R₁, R₂, R₃, R₄, -(CH₂)ₙ- groups are branched or straight chains, and may contain C, H, O, S, N, and other atoms and may contain single, double, triple bonds and ring systems.

9. Processes for the preparation of compounds of the general formula (2, 3, 5, 6, 7, 8, 9, 10, 11, 12, 16, 17, 22, 23, 24, 25, 26, and 27) 'Structure 2, 3, 5, 6, 7, 8, 9, 10, 11, 12, 16, 17, 22, 23, 24, 25, 26, and 27' according to claims 1 to 8, wherein the compounds can be prepared from retinoic acids and related compounds, by reaction with compounds of the general formula (28) 'Structure 28' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris(dimethylamino)phosphonium hexafluorophosphate, et al., 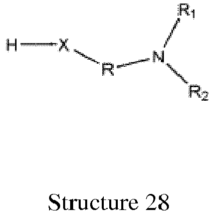 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₆COR₄, SOR₄, SR₄, PO₃R₆R₆, C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₄COR₅, SOR₅, SR₅, PO₃R₅R₄, C₁₋₆ alkyl, C₁₋₆ alkyloxy; C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₄R₅, aryl or heteroaryl residues, or other ring systems; X represents O, S, or NH. The double bonds on the side chain may have Z or E configuration. All R, R₁, R₂, R₃, R₄, -(CH₂)ₙ- groups are branched or straight chains, and may contain C, H, O, S, N, and other atoms and may contain single, double, triple bonds and ring systems.

10. Processes for the preparation of compounds of the general formula (4 , 13, 14, 15, 20, and 21) 'Structure 4 , 13, 14, 15, 20, or 21' according to claims 3, 6 and 7, wherein the compounds can be prepared from retinol and related compounds, by reaction with compounds of the general formula (29) 'Structure 29', 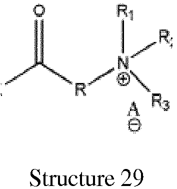 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₆COR₄, SOR₄, SR₄, PO₃R₆R₆ , C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₄COR₅, SOR₅, SR₅, PO₃R₅R₄, C₁₋₆ alkyl, C₁₋₆ alkyloxy; C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₄R₅, aryl or heteroaryl residues, or other ring systems; Z represents F, Cl, Br, or I; A⁻ represents Cl⁻, Br⁻, F⁻, I⁻, AcO⁻, citrate, or any negative ions. The double bonds on the side chain may have Z or E configuration. All R, R₁, R₂, R₃, R₄, -(CH₂)ₙ- groups are branched or straight chains, and may contain C, H, O, S, N, and other atoms and may contain single, double, triple bonds and ring systems.

11. Processes for the preparation of compounds of the general formula (2, 3, 5, 6, 7, 8, 9, 10, 11, 12, 16, 17, 22, 23, 24, 25, 26, and 27) 'Structure 2, 3, 5, 6, 7, 8, 9, 10, 11, 12, 16, 17, 22, 23, 24, 25, 26, and 27' according to claims 1, 2 and 4 to 8, wherein the compounds can be prepared from metal salts, organic base salts , or immobilized base salts of retinoic acids or related compounds when X represents O, by reaction with compounds of the general formula (30) 'Structure 30', 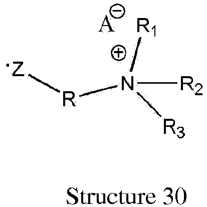 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₆R₆', aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl residues or taken together are -(CH₂)ₙ-, wherein n=2, 3, 4, 5, 6, 7, 8, 9, 10,..., and any CH₂ may be replaced with O, S, CH=CH, C≡C, CR₆R₆', aryl or heteroaryl residues, or other ring systems; R₃ represents H, one of any alkyl, alkyloxy, alkenyl or alkynyl residues having 1 to 12 carbon atoms, aryl or heteroaryl residues, wherein, any CH₂ may be replaced with O, S, CH=CH, C≡C, CR₆R₆', aryl or heteroaryl residues, or other ring systems; R₄ represents H, OH, Cl, Br, F, I, NO₂, CN, SO₂R₅, COR₅, COOR₅, NR₆COR₅, SOR₅, SR₅, PO₃R₆R₆', C₁₋₆ alkyl, C₁₋₆ alkyloxy; C₁₋₆ perfluoroalkyl, C₁₋₆ alkeny, C₁₋₆ alkyny, or C₁₋₆ alkyl halide; R₅ represents H, OH, Cl, Br, F, I, NO₂, CN, SO₂R₄, COR₄, COOR₄, NR₆COR₄, SOR₄, SR₄, PO₃R₆R₆', C₁₋₆ alkyl, C₁₋₆ alkyloxy, C₁₋₆ perfluoroalkyl, C₁₋₆ alkeny, C₁₋₆ alkyny, or C₁₋₆ alkyl halide; R₆ and R₆' taken alone are same or different and are H, Cl, Br, F, I, OH, NO₂, CN, SO₂R₅, COR₅, COOR₅, NR₄COR₅, SOR₅, SR₅, PO₃R₅R₄, C₁₋₆ alkyl, C₁₋₆ alkyloxy; C₁₋₆ alkeny, C₁₋₆ alkyny, or alkyl halide or taken together are oxygen (=O) or -(CH₂)ₙ-, wherein n=2, 3, 4, 5, 6, 7, 8, 9, 10,..., and any CH₂ may be replaced with O, S, CH=CH, C≡C, CR₄R₅, aryl or heteroaryl residues, or other ring systems; Z represents halogen, or p-toluenesulphonyl; A⁻ represents Cl⁻, Br⁻ , F⁻, I⁻, AcO⁻, citrate, or any negative ions. The double bonds on the side chain may have Z or E configuration. All R, R₁, R₂, R₃, R₄, -(CH₂)ₙ- groups are branched or straight chains, and may contain C, H, O, S, N, and other atoms and may contain single, double, triple bonds and ring systems.

12. Compounds of the general formulas (2 to 27) 'Structure 2 to 27' or a composition comprising of at least one compound of the general formulas (2 to 27)' Structure 2 to 27', as an active ingredient, according to claims 1 to 8 for use as an active ingredient that can be administered orally or transdermally, for treating any retinoids and retinoid-like compounds-treatable conditions in humans or animals., wherein the retinoids and retinoid-like compounds-treatable conditions include, but are not limited to acne, acne scarring, psoriasis, ichthyosis, eczema, keratinization disorders, precancerous lesions, chemoprophylaxis, warts, sarcoidosis, treating photoaged skin, preventing photoaged skin, treating chronologically aged skin, hair loss, and cancers.

13. Use of the compounds of the general formulas (2 to 27) 'Structure 2 to 27' according to claims 1 to 8, for treating any retinoids and retinoid-like compounds-treatable conditions in humans or animals by administering transdermally (topically) to any part of body (in the form of a solution, spray, lotion, ointment, emulsion or gel) to deliver therapeutically effective plasma levels of the compounds of the general formulas (2 to 27) 'Structure 2 to 27', as an active ingredient.

14. Use of the compounds of the general formulas (2 to 27) 'Structure 2 to 27' according to claims 1 to 8, for topically treating acne, acne scarring, psoriasis, ichthyosis, eczema, keratinization disorders, precancerous lesions, chemoprophylaxis, warts, sarcoidosis, treating photoaged skin, preventing photoaged skin, treating chronologically aged skin, hair loss, and cancers in humans or animals by administering to the particular site of disease a therapeutically effective amount of the compounds of the general formulas (2 to 27) 'Structure 2 to 27', as an active ingredient.

15. Transdermal therapeutic application systems of compounds of general formulas (2 to 27)' Structure 2 to 27', as an active ingredient, according to Claims 1 to 8, for treating any retinoids and retinoid-like compounds-treatable conditions in humans or animals; optionally wherein the systems are a bandage or a patch comprising of one active substance-containing matrix layer and an impermeable backing layer; preferably wherein the system is an active substance reservoir, which has a permeable bottom facing the skin.
